(19) Europäisches Patentamt
European Patent Office
Office européen des brevets

(11) Veröffentlichungsnummer: **0 646 597 A1**

(12) **EUROPÄISCHE PATENTANMELDUNG**

(21) Anmeldenummer: **94113560.0**

(51) Int. Cl.[6]: **C07K 5/068**, A61K 38/02

(22) Anmeldetag: **31.08.94**

(30) Priorität: **14.09.93 DE 4331134**

(43) Veröffentlichungstag der Anmeldung:
**05.04.95 Patentblatt 95/14**

(84) Benannte Vertragsstaaten:
**AT BE CH DE DK ES FR GB GR IE IT LI LU MC NL PT SE**

(71) Anmelder: **BAYER AG**

**D-51368 Leverkusen (DE)**

(72) Erfinder: **Häbich, Dr. Dieter**
**Krummacherstrasse 82**
**D-42115 Wuppertal (DE)**
Erfinder: **Schulze, Dr. Thomas-J.**
**Jakob-Böhme-Strasse 11**
**D-51065 Köln (DE)**
Erfinder: **Reefschläger, Jürgen**
**Kranichweg 14**
**D-42111 Wuppertal (DE)**
Erfinder: **Hansen, Dr. Jutta**
**Pahlkestrasse 98**
**D-42115 Wuppertal (DE)**
Erfinder: **Neumann, Rainer**
**Olefstrasse 11**
**D-50937 Köln (DE)**
Erfinder: **Streissle, Gert**
**Gellertweg 22**
**D-42115 Wuppertal (DE)**
Erfinder: **Paessens, Dr. Arnold**
**Stresemannstrasse 51**
**D-42781 Haan (DE)**

(54) **Neue antiviral wirksame Pseudopeptide.**

(57) Die vorliegende Erfindung betrifft jetzt neue antiviral wirksame Pseudopeptide der allgemeinen Formel (I)

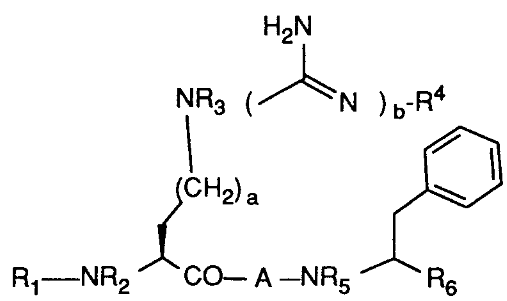

(I)

in welcher
die Substituenten die in der Beschreibung angegebene Bedeutung haben, ein Verfahren zu ihrer Herstellung sowie ihre Verwendung als antivirale Mittel, insbesondere gegen Cytomegalieviren.

EP 0 646 597 A1

Die vorliegende Erfindung betrifft neue antiviral wirksame Pseudopeptide, Verfahren zu ihrer Herstellung sowie ihre Verwendung als antivirale Mittel, insbesondere gegen Cytomegalieviren.

Aus den Publikationen J. Antibiot. 44, 1019 (1991), FEBS Letters 3, 253 (1993) sowie in der Patentanmeldung WO 92/22570 werden Peptidaldehyde als Inhibitoren der HIV-Protease bzw. von Picornavirus-Proteasen beschrieben. Desweiteren wurden Peptidaldehyde als Inhibitoren von Serinproteasen beschrieben [US 5 153 176; EP 516 877].

Als Verbindungsklassen mit anti-Cytomegalievirusaktivität sind verschiedene Nucleosid- und Nucleotidanaloga, Anthrachinon-Derivate, Cobalt-Komplexe, Macrolide und Acylpeptide [EP 488 041] bekannt.

Die vorliegende Erfindung betrifft jetzt neue antiviral wirksame Pseudopeptide der allgemeinen Formel (I)

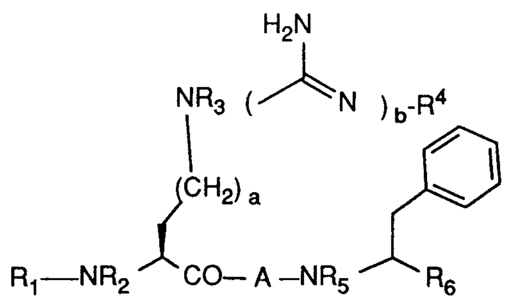

(I)

in welcher

a für eine Zahl 1, 2 oder 3 steht,

b für eine Zahl 0 oder 1 steht,

$R^1$ für Wasserstoff oder für eine Aminoschutzgruppe steht, oder für einen Rest der Formel $R^7$-$NR^8$-CO-, $R^9$-$(CH_2)_c$-CO-, $R^{10}$-$(CH_2)_d$-O-CO oder für einen Rest der Formel -$SO_2$-$R^{11}$ steht,

worin

$R^7$ Cycloalkyl mit 3 bis 6 Kohlenstoffatomen bedeutet oder geradkettiges oder verzweigtes Alkyl mit bis zu 18 Kohlenstoffatomen bedeutet, das gegebenenfalls durch Hydroxy, geradkettiges oder verzweigtes Alkoxy mit bis zu 4 Kohlenstoffatomen, Halogen, Trifluormethyl, Trifluormethoxy, Cycloalkyl mit 3 bis 6 Kohlenstoffatomen oder durch Aryl mit 6 bis 10 Kohlenstoffatomen substituiert ist, das seinerseits bis zu 2-fach gleich oder verschieden durch Carboxy, Cyano, Hydroxy, Halogen, Perhalogenalkyl mit bis zu 5 Kohlenstoffatomen oder durch geradkettiges oder verzweigtes Acyl, Alkoxy oder Alkoxycarbonyl mit jeweils bis zu 6 Kohlenstoffatomen substituiert sein kann, oder

Alkyl gegebenenfalls durch eine Gruppe der Formel -$CO_2R^{12}$ substituiert ist,

worin

$R^{12}$ Wasserstoff oder geradkettiges oder verzweigtes Alkyl oder Alkenyl mit jeweils bis zu 8 Kohlenstoffatomen bedeutet, die gegebenenfalls durch Phenyl substituiert sind,

oder

$R^7$ Aryl mit 6 bis 10 Kohlenstoffatomen bedeutet, das gegebenenfalls bis zu 3-fach gleich oder verschieden durch Carboxy, Amino, Halogen, Hydroxy, Cyano, Perhalogenalkyl mit bis zu 5 Kohlenstoffatomen oder durch geradkettiges oder verzweigtes Acyl, Alkoxy, Vinylalkoxycarbonyl, Alkoxycarbonyl oder mit jeweils bis zu 6 Kohlenstoffatomen substituiert ist, das seinerseits durch geradkettiges oder verzweigtes Alkoxy mit bis zu 6 Kohlenstoffatomen substituiert ist, oder

einen Aminosäurerest der Formel

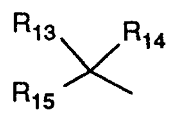

bedeutet,
worin

$R^{13}$ und $R^{14}$ gleich oder verschieden sind und Wasserstoff oder Methyl bedeuten, oder

$R^{13}$ und $R^{14}$ gemeinsam einen 5- oder 6-gliedrigen gesättigten carbocyclischen Ring bilden,
oder

$R^{13}$ Wasserstoff oder Methyl bedeutet
und

$R^{14}$ Cycloalkyl mit 3 bis 8 Kohlenstoffatomen oder Aryl mit 6 bis 10 Kohlenstoffatomen oder Wasserstoff bedeutet, oder
geradkettiges oder verzweigtes Alkyl mit bis zu 8 Kohlenstoffatomen bedeutet,
wobei das Alkyl gegebenenfalls durch Methylthio, Hydroxy, Mercapto,
Guanidyl oder durch eine Gruppe der Formel $-NR^{16}R^{17}$ oder $R^{18}$-OC- substituiert ist,
worin

$R^{16}$ und $R^{17}$ unabhängig voneinander Wasserstoff, geradkettiges oder verzweigtes Alkyl mit bis zu 8 Kohlenstoffatomen oder Phenyl bedeuten,
und

$R^{18}$ Hydroxy, Benzyloxy, Alkoxy mit bis zu 6 Kohlenstoffatomen oder die oben aufgeführte Gruppe $-NR^{16}R^{17}$ bedeutet,

$R^{15}$ geradkettiges oder verzweigtes Alkyl mit bis zu 8 Kohlenstoffatomen bedeutet, das gegebenenfalls durch Hydroxy oder geradkettiges oder verzweigtes Alkoxy mit bis zu 6 Kohlenstoffatomen substituiert ist, oder
Carboxy, Allyloxycarbonyl, geradkettiges oder verzweigtes Alkoxycarbonyl mit bis zu 8 Kohlenstoffatomen oder Benzyloxycarbonyl bedeutet,
oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^{16}R^{17}$ substituiert ist,
worin

$R^{16}$ und $R^{17}$ die oben angegebene Bedeutung haben,
oder das Alkyl gegebenenfalls durch einen 5- bis 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,
oder

$R^7$ einen Rest der Formel

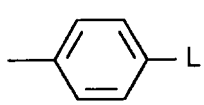

bedeutet,
worin

L Phenyl oder Pyridyl bedeutet

$R^8$ Wasserstoff, geradkettiges oder verzweigtes Alkyl mit bis zu 6 Kohlenstoffatomen oder eine Aminoschutzgruppe bedeutet,

$R^9$ geradkettiges oder verzweigtes Alkyl mit bis zu 8 Kohlenstoffatomen, welches durch Pyridyl substituiert sein kann, bedeutet, oder Aryloxy oder Aryl mit jeweils 6 bis 10 Kohlenstoffatomen, Indolyl, Chinolyl, Chinoxalinyl, Isochinolyl oder einen 5- bis 7-gliedrigen, gesättigten oder ungesättigten Heterocyclus mit bis zu 3 Heteroatomen aus der Reihe S, N oder O bedeutet, wobei die Cyclen bis zu 3-fach gleich oder verschieden durch Carboxy, Cyano, Hydroxy, Halogen, Amino, Nitro, Methylamino, Perhalogenalkyl mit bis zu 5 Kohlenstoffatomen oder durch geradkettiges oder verzweigtes Alkyl, Acyl, Alkoxy oder Alkoxycarbonyl mit jeweils bis zu 6 Kohlenstoffatomen substituiert sein können,
oder Aryl gegebenenfalls auch durch einen 5- bis 7-gliedrigen, gesättigten oder ungesättigten Heterocyclus mit bis zu 3 Heteroatomen aus der Reihe S, N oder O

substituiert ist, das seinerseits durch Phenyl substituiert sein kann, oder

$R^9$ einen Rest der Formel

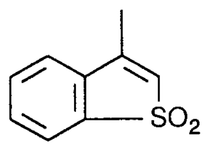
,
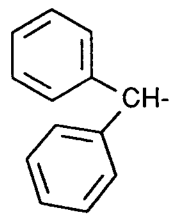
,

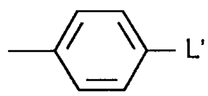
oder
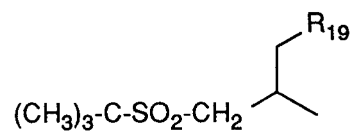

bedeutet,
worin

L' die oben angegebene Bedeutung von L hat und mit dieser gleich oder verschieden ist,

$R^{19}$ Phenyl oder Naphthyl bedeutet,

c eine Zahl 0, 1, 2 oder 3 bedeutet,

d eine Zahl 0, 1, 2 oder 3 bedeutet,

$R^{10}$ die oben angegebene Bedeutung von $R^9$ hat und mit dieser gleich oder verschieden ist,

$R^{11}$ Methyl, Phenyl oder Naphthyl bedeutet, das gegebenenfalls bis zu 4-fach gleich oder verschieden durch Methyl oder Methoxy substituiert ist, oder einen Rest der Formel

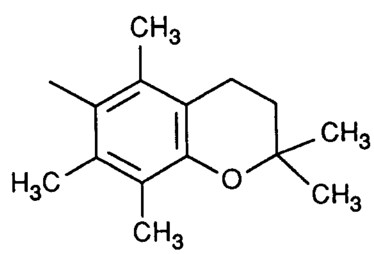

bedeutet,

$R^2$, $R^3$ und $R^5$ gleich oder verschieden sind und für Wasserstoff, geradkettiges oder verzweigtes Alkyl mit bis zu 4 Kohlenstoffatomen oder für eine Aminoschutzgruppe stehen,

$R^4$ für Wasserstoff, für Nitro, für eine Aminoschutzgruppe oder für einen Rest der Formel $-SO_2-R^{20}$ steht,
worin

$R^{20}$ die oben angegebene Bedeutung von $R^{11}$ hat und mit dieser gleich oder verschieden ist,

A für eine Bindung oder für einen Rest der Formel

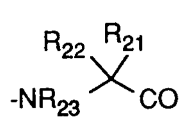

steht,
worin

| | |
|---|---|
| $R^{21}$ und $R^{22}$ | gleich oder verschieden sind und die oben angegebene Bedeutung von $R^{13}$ und $R^{14}$ haben und mit dieser gleich oder verschieden sind, |
| $R^{23}$ | die oben angegebene Bedeutung von $R^2$, $R^3$ oder $R^5$ hat und mit dieser gleich oder verschieden ist, |
| $R^6$ | für Formyl oder Carboxy steht oder für geradkettiges oder verzweigtes Alkoxycarbonyl mit bis zu 8 Kohlenstoffatomen steht, oder für einen Rest der Formel $-CH_2-OR^{24}$ oder $-CH(OR^{25})_2$ steht, worin |
| $R^{24}$ und $R^{25}$ | gleich oder verschieden sind und Wasserstoff, geradkettiges oder verzweigtes Alkyl mit bis zu 6 Kohlenstoffatomen oder eine Hydroxyschutzgruppe bedeuten, |

und deren Salze,
mit der Maßgabe, daß A nicht für Valin stehen darf.

Die erfindungsgemäßen Verbindungen der allgemeinen Formel (I) können auch in Form ihrer Salze vorliegen. Im allgemeinen seien hier Salze mit organischen und anorganischen Basen oder Säuren genannt.

Zu den Säuren, die addiert werden können, gehören vorzugsweise Halogenwasserstoffsäuren, wie z.B. die Fluorwasserstoffsäure, Chlorwasserstoffsäure und die Bromwasserstoffsäure, insbesondere die Fluor- und Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Hydroxyschutzgruppe im Rahmen der oben angegebenen Definition steht im allgemeinen für eine Schutzgruppe aus der Reihe: tert.Butoxydiphenylsilyl, Trimethylsilyl, Triethylsilyl, Triisopropylsilyl, tert.Butyldimethylsilyl, tert.-Butyldiphenylsilyl, Triphenylsilyl, Trimethylsilylethoxycarbonyl, Benzyl, Benzyloxycarbonyl, 2-Nitrobenzyl, 4-Nitrobenzyl, 2-Nitrobenzyloxycarbonyl, 4-Nitrobenzyloxycarbonyl, tert. Butyloxycarbonyl, Allyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methylthiomethyl, Methoxyethoxymethyl, [2-(Trimethylsilyl)ethoxy]methyl, 2-(Methylthiomethoxy)ethoxycarbonyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, Benzoyl, Benzyl oder Methylbenzyl.

Aminoschutzgruppe im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.-Butoxycarbonyl, Allyloxycarbonyl, Vinyloxycarbonyl, 2-Nitrobenzyloxycarbonyl, 3,4,5-Trimethoxybenzyloxycarbonyl, Cyclohexyloxycarbonyl, 1,1-Dimethylethoxycarbonyl, Adamantylcarbonyl, Phthaloyl, 2,2,2-Trichiorethoxycarbonyl, 2,2,2-Trichlortertbutoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl.

Heterocyclus steht im allgemeinen für eine 5- bis 7-gliedrigen, bevorzugt 5- bis 6-gliedrigen, gesättigten oder ungesättigten Ring, der als Heteroatome bis zu 3 Sauerstoff-, Schwefel- und/oder Stickstoffatome enthalten kann. Bevorzugt sind 5- und 6-gliedrige Ringe mit einem Sauerstoff-, Schwefel- und/oder bis zu 3 Stickstoffatomen. Besonders bevorzugt werden genannt: Pyrrolyl, Pyrazolyl, Pyridyl, Pyrimidyl, Pyrazinyl, Pyridazinyl, Thiazolyl, Oxazolyl, Imidazolyl, Isoxazolyl, Pyrrolidinyl, Piperidinyl, Piperazinyl, Tetrazolyl oder Morpholinyl.

Die erfindungsgemäßen Verbindungen der allgemeinen Formel (I) besitzen, wie die Verbindung der allgemeinen Formel (II)

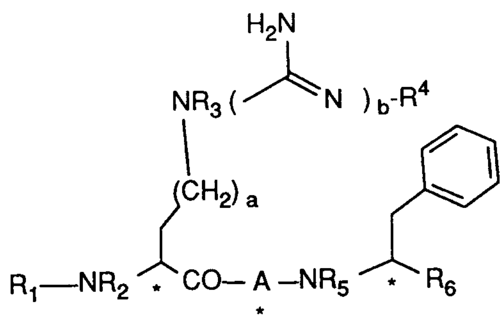

(II)

zeigt, mindestens 3 asymmetrische Kohlenstoffatome (*). Sie können unabhängig voneinander in der D- oder L-Form, und R- oder S-Konfiguration vorliegen. Die Erfindung umfaßt die optischen Antipoden ebenso wie die Isomerengemische oder Racemate.

Die erfindungsgemäßen Verbindungen der allgemeinen Formel (I) können in stereoisomeren Formen existieren, beispielsweise entweder wie Bild und Spiegelbild (Enantiomere), oder die sich nicht wie Bild und Spiegelbild (Diastereomere) verhalten, beziehungsweise als Diastereomerengemisch vorliegen. Die Erfindung betrifft sowohl die Antipoden, Racemformen, Diastereomerengemische sowie die reinen Isomeren. Die Racemformen lassen sich ebenso wie die Diastereomerengemische in bekannter Weise in die stereoisomer einheitlichen Bestandteile trennen.

Die Trennung in die stereoisomer einheitlichen Verbindungen erfolgt beispielsweise über eine chromatographische Racematspaltung von diastereomeren Estern und Amiden oder an optisch aktiven Phasen. Außerdem ist eine Kristallisation von diastereomeren Salzen möglich.

Bevorzugt sind Verbindungen der allgemeinen Formel (I),
in welcher

a für eine Zähl 2 oder 3 steht,

b für eine Zahl 0 oder 1 steht,

$R^1$ für Wasserstoff, tert.Butoxycarbonyl (Boc), Benzyloxycarbonyl (Z) oder 9-Fluorenylmethoxycarbonyl (FMOC) steht, oder
für einen Rest der Formel $R^7$-$NR^8$-CO-, $R^9$-$(CH_2)_c$-CO-, $R^{10}$-$(CH_2)_d$-O-CO oder für einen Rest der Formel -$SO_2$-$R^{11}$ steht,
worin

$R^7$ Cyclopentyl oder Cyclohexyl bedeutet oder geradkettiges oder verzweigtes Alkyl mit bis zu 16 Kohlenstoffatomen bedeutet, das gegebenenfalls durch Hydroxy, Methoxy, Fluor, Chlor, Brom, Trifluormethyl, Trifluormethoxy, Cyclopentyl, Cyclohexyl oder Phenyl substituiert ist, das seinerseits bis zu 2-fach gleich oder verschieden durch Carboxy, Cyano, Hydroxy, Fluor, Chlor, Brom, Perhalogenalkyl mit bis zu 4 Kohlenstoffatomen oder durch geradkettiges oder verzweigtes Acyl, Alkoxy oder Alkoxycarbonyl mit jeweils bis zu 4 Kohlenstoffatomen substituiert sein kann, oder
Alkyl gegebenenfalls durch eine Gruppe der Formel -$CO_2R^{12}$ substituiert ist,
worin

$R^{12}$ Wasserstoff oder geradkettiges oder verzweigtes Alkyl oder Alkenyl mit jeweils bis zu 6 Kohlenstoffatomen bedeutet, die gegebenenfalls durch Phenyl substituiert sind,
oder

$R^7$ Phenyl oder Naphthyl bedeutet, das gegebenenfalls bis zu 3-fach gleich oder verschieden durch Carboxy, Amino, Fluor, Chlor, Brom, Hydroxy, Cyano, Perhalogenalkyl mit bis zu 4 Kohlenstoffatomen oder durch geradkettiges oder verzweigtes Acyl, Alkoxy, Vinylalkoxycarbonyl oder Alkoxycarbonyl mit jeweils bis zu 5 Kohlenstoffatomen substituiert ist, das seinerseits durch geradkettiges oder verzweigtes Alkoxy mit bis zu 6 Kohlenstoffatomen substituiert ist, oder
einen Aminosäurerest der Formel

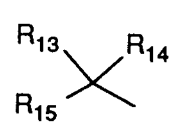

bedeutet,
worin

$R^{13}$ und $R^{14}$ gleich oder verschieden sind und Wasserstoff oder Methyl bedeuten, oder

$R^{13}$ und $R^{14}$ gemeinsam einen Cyclopentyl- oder Cyclohexylring bilden,
oder

$R^{13}$ Wasserstoff oder Methyl bedeutet
und

$R^{14}$ Cyclopropyl, Cyclopentyl, Cyclohexyl, Phenyl oder Wasserstoff bedeutet, oder geradkettiges oder verzweigtes Alkyl mit bis zu 6 Kohlenstoffatomen bedeutet, wobei das Alkyl gegebenenfalls durch Methylthio, Hydroxy, Mercapto, Guanidyl oder durch eine Gruppe der Formel $-NR^{16}R^{17}$ oder $R^{18}$-OC- substituiert ist,
worin

$R^{16}$ und $R^{17}$ unabhängig voneinander Wasserstoff, geradkettiges oder verzweigtes Alkyl mit bis zu 6 Kohlenstoffatomen oder Phenyl bedeuten,
und

$R^{18}$ Hydroxy, Benzyloxy, Alkoxy mit bis zu 6 Kohlenstoffatomen oder die oben aufgeführte Gruppe $-NR^{16}R^{17}$ bedeutet,

oder das Alkyl gegebenenfalls durch Cyclopropyl, Cyclopentyl, Cyclohexyl oder Phenyl substituiert ist, das seinerseits durch Hydroxy, Fluor, Chlor, Brom, Nitro, Alkoxy mit bis zu 8 Kohlenstoffatomen oder durch die Gruppe $-NR^{16}R^{17}$ substituiert ist,
worin

$R^{16}$ und $R^{17}$ die oben angegebene Bedeutung haben,

oder das Alkyl gegebenenfalls durch Imidazolyl oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,

$R^{15}$ geradkettiges oder verzweigtes Alkyl mit bis zu 6 Kohlenstoffatomen bedeutet, das gegebenenfalls durch Hydroxy oder geradkettiges oder verzweigtes Alkoxy mit bis zu 4 Kohlenstoffatomen substituiert ist, oder
Carboxy, Allyloxycarbonyl, geradkettiges oder verzweigtes Alkoxycarbonyl mit bis zu 6 Kohlenstoffatomen oder Benzyloxycarbonyl bedeutet,

$R^7$ einen Rest der Formel

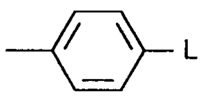

bedeutet,
worin

L Phenyl oder Pyridyl bedeutet

$R^8$ Wasserstoff, geradkettiges oder verzweigtes Alkyl mit bis zu 4 Kohlenstoffatomen, tert.Butoxycarbonyl (Boc) oder Benzyloxycarbonyl (Z) bedeutet,

$R^9$ geradkettiges oder verzweigtes Alkyl mit bis zu 6 Kohlenstoffatomen, welches durch Pyridyl substituiert sein kann, bedeutet, oder Phenoxy, Phenyl, Naphthyl, Indolyl, Chinolyl, Chinoxalinyl, Isochinolyl, Pyridyl, Pyrazinyl, Pyrimidyl, Triazolyl oder Imidazolyl bedeutet, wobei die Cyclen gegebenenfalls bis zu 3-fach gleich oder verschieden durch Nitro, Carboxy, Cyano, Hydroxy, Fluor, Chlor, Brom, Perhalogenalkyl mit bis zu 4 Kohlenstoffatomen oder durch geradkettiges oder verzweigtes Alkyl, Acyl, Alkoxy oder Alkoxycarbonyl mit jeweils bis zu 4 Kohlenstoffatomen substituiert sind, oder Phenyl gegebenenfalls durch Pyridyl oder Triazolyl substituiert ist, wobei diese wiederum durch Phenyl substituiert sein können,
oder

$R^9$ einen Rest der Formel

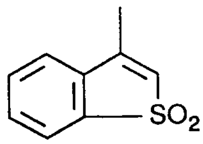 , 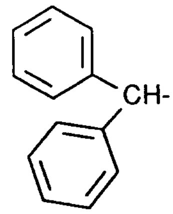 ,

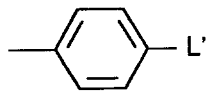 oder 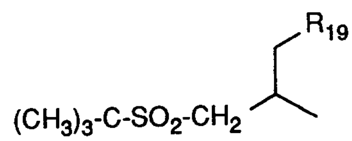

bedeuten,
worin

L' die oben angegebene Bedeutung von L hat und mit dieser gleich oder verschieden ist,

$R^{19}$ Phenyl oder Naphthyl bedeutet,

c eine Zahl 0, 1, 2 oder 3 bedeutet,

d eine Zahl 0, 1 oder 2 bedeutet,

$R^{10}$ die oben angegebene Bedeutung von $R^9$ hat und mit dieser gleich oder verschieden ist,

$R^{11}$ Methyl oder Phenyl bedeutet, das gegebenenfalls bis zu 4-fach gleich oder verschieden durch Methyl oder Methoxy substituiert ist, oder einen Rest der Formel

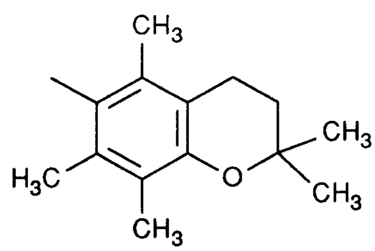

bedeutet,

$R^2$, $R^3$ und $R^5$ gleich oder verschieden sind und Boc, Wasserstoff, Methyl, Ethyl, Benzyloxycarbonyl oder tert.Butyl bedeuten,

$R^4$ für Wasserstoff, Nitro, Benzyloxycarbonyl, tert.Butoxycarbonyl oder für einen Rest der Formel $-SO_2R^{20}$ steht,
worin

$R^{20}$ die oben angegebene Bedeutung von $R^{11}$ hat und mit dieser gleich oder verschieden ist,

A für eine Bindung oder für einen Rest der Formel

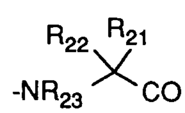

steht,
worin

$R^{21}$ und $R^{22}$ gleich oder verschieden sind und die oben angegebene Bedeutung von $R^{13}$ und $R^{14}$ haben und mit dieser gleich oder verschieden sind,

$R^{23}$ die oben angegebene Bedeutung von $R^2$, $R^3$ oder $R^5$ hat und mit dieser gleich oder

verschieden ist,

$R^6$ für Formyl oder Carboxy steht oder
für geradkettiges oder verzweigtes Alkoxycarbonyl mit bis zu 6 Kohlenstoffatomen steht, oder
für einen Rest der Formel $-CH_2-OR^{24}$ oder $-CH(OR^{25})_2$ steht,
worin

$R^{24}$ und $R^{25}$ gleich oder verschieden sind und Wasserstoff, geradkettiges oder verzweigtes Alkyl mit bis zu 4 Kohlenstoffatomen, Acetyl oder Benzyl bedeuten,

und deren Salze,
mit der Maßgabe, daß A nicht für Valin stehen darf.

Besonders bevorzugt sind Verbindungen der allgemeinen Formel (I),
in welcher

a für eine Zahl 2 oder 3 steht,

b für eine Zahl 0 oder 1 steht,

$R^1$ für Wasserstoff, tert.Butoxycarbonyl (Boc) oder Benzyloxycarbonyl (Z) steht, oder
für einen Rest der Formel $R^7-NR^8-CO-$, $R^9-(CH_2)_c-CO-$, $R^{10}-(CH_2)_d-O-CO$ oder für einen Rest der Formel $-SO_2-R^{11}$ stehen,
worin

$R^7$ Cyclopentyl oder Cyclohexyl bedeutet oder geradkettiges oder verzweigtes Alkyl mit bis zu 14 Kohlenstoffatomen bedeutet, das gegebenenfalls durch Hydroxy, Methoxy, Fluor, Trifluormethyl, Trifluormethoxy, Cyclohexyl oder Phenyl substituiert ist, das gegebenenfalls durch eine Gruppe der Formel $-CO_2R^{12}$ substituiert ist,
worin

$R^{12}$ Wasserstoff oder geradkettiges oder verzweigtes Alkyl oder Alkenyl mit jeweils bis zu 4 Kohlenstoffatomen oder Benzyl bedeutet,
oder

$R^7$ Phenyl bedeutet, das gegebenenfalls bis zu 3-fach gleich oder verschieden durch Carboxy, Fluor, Hydroxy, Cyano, Trifluormethyl, Amino oder durch geradkettiges oder verzweigtes Acyl, Alkoxy, Vinylalkoxycarbonyl oder Alkoxycarbonyl mit jeweils bis zu 4 Kohlenstoffatomen substituiert ist, das seinerseits durch geradkettiges oder verzweigtes Alkoxy mit bis zu 4 Kohlenstoffatomen substituiert ist, oder
einen Aminosäurerest der Formel

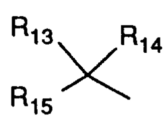

bedeutet,
worin

$R^{13}$ und $R^{14}$ gleich oder verschieden sind und Wasserstoff oder Methyl bedeuten, oder

$R^{13}$ und $R^{14}$ gemeinsam einen Cyclopentyl- oder Cyclohexylring bilden,
oder

$R^{13}$ Wasserstoff oder Methyl bedeutet
und

$R^{14}$ Cyclopropyl, Cyclopentyl, Cyclohexyl, Phenyl oder Wasserstoff bedeutet, oder
geradkettiges oder verzweigtes Alkyl mit bis zu 6 Kohlenstoffatomen bedeutet,
wobei das Alkyl gegebenenfalls durch Methylthio, Hydroxy, Mercapto,
Guanidyl oder durch eine Gruppe der Formel $-NR^{16}R^{17}$ oder $R^{18}-OC-$ substituiert ist,
worin

$R^{16}$ und $R^{17}$ unabhängig voneinander Wasserstoff, geradkettiges oder verzweigtes Alkyl mit bis zu 6 Kohlenstoffatomen oder Phenyl bedeuten,
und

$R^{18}$ Hydroxy, Benzyloxy, Alkoxy mit bis zu 4 Kohlenstoffatomen oder die oben aufgeführte Gruppe $-NR^{16}R^{17}$ bedeutet,

oder das Alkyl gegebenenfalls durch Cyclopropyl, Cyclopentyl, Cyclohexyl oder Phenyl substituiert ist, das seinerseits durch Hydroxy, Fluor, Chlor, Brom, Nitro, Alkoxy

mit bis zu 6 Kohlenstoffatomen oder durch die Gruppe $-NR^{16}R^{17}$ substituiert ist, worin

$R^{16}$ und $R^{17}$ die oben angegebene Bedeutung haben,

oder das Alkyl gegebenenfalls durch Imidazolyl oder Indolyl substituiert ist, worin die entsprechenden -NH-Funktionen gegebenenfalls durch Alkyl mit bis zu 4 Kohlenstoffatomen, tert. Butoxycarbonyl oder Benzyloxycarbonyl geschützt sind,

$R^{15}$ geradkettiges oder verzweigtes Alkyl mit bis zu 4 Kohlenstoffatomen bedeutet, das gegebenenfalsl durch Hydroxy oder geradkettiges oder verzweigtes Alkoxy mit bis zu 3 Kohlenstoffatomen substituiert ist, oder
Carboxy, Allyloxycarbonyl, geradkettiges oder verzweigtes Alkoxycarbonyl mit bis zu 4 Kohlenstoffatomen oder Benzyloxycarbonyl bedeutet,

oder

$R^7$ einen Rest der Formel

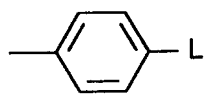

bedeutet,
worin

L Phenyl oder Pyridyl bedeutet

$R^8$ Wasserstoff, Methyl, Ethyl oder tert.Butyl, bedeutet,

$R^9$ geradkettiges oder verzweigtes Alkyl mit bis zu 6 Kohlenstoffatomen, welches durch Pyridyl substituiert sein kann, Phenoxy, Phenyl, Naphthyl, Indolyl, Chinolyl, Chinoxalinyl, Isochinolyl, Pyridyl, Pyrazinyl, Pyrimidyl, Triazolyl oder Imidazolyl bedeutet, wobei die Cyclen gegebenenfalls bis zu 3-fach gleich oder verschieden durch Nitro, Carboxy, Cyano, Hydroxy, Fluor, Chlor, Brom, Perhalogenalkyl mit bis zu 4 Kohlenstoffatomen oder durch geradkettiges oder verzweigtes Alkyl, Acyl, Alkoxy oder Alkoxycarbonyl mit jeweils bis zu 4 Kohlenstoffatomen substituiert sind, oder
Phenyl gegebenenfalls durch Pyridyl oder Triazolyl substituiert ist, wobei diese wiederum durch Phenyl substituiert sein können,

oder

$R^9$ einen Rest der Formel

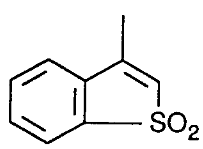 , 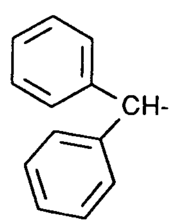 ,

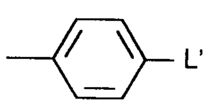 oder 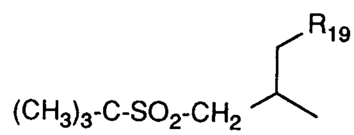

bedeutet,
worin

L' die oben angegebene Bedeutung von L hat und mit dieser gleich oder verschieden ist,

$R^{19}$ Phenyl oder Naphthyl bedeutet,

c eine Zahl 0, 1, 2 oder 3 bedeutet,

d eine Zahl 0, 1 oder 2 bedeutet,

| | |
|---|---|
| $R^{10}$ | die oben angegebene Bedeutung von $R^9$ hat und mit dieser gleich oder verschieden ist, |
| $R^{11}$ | Methyl oder Phenyl bedeutet, das gegebenenfalls bis zu 4-fach gleich oder verschieden durch Methyl oder Methoxy substitiert ist, oder einen Rest der Formel |

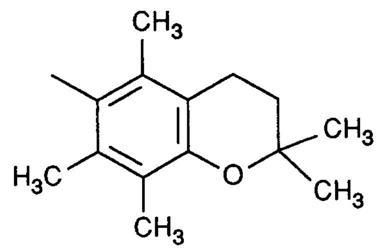

bedeutet,

| | |
|---|---|
| $R^2$, $R^3$ und $R^5$ | gleich oder verschieden sind und Boc, Wasserstoff, Methyl, Ethyl, Benzyloxycarbonyl oder tert.Butyl bedeuten, |
| $R^4$ | für Wasserstoff, Nitro, Benzyloxycarbonyl, tert.Butoxycarbonyl oder für einen Rest der Formel $-SO_2R^{20}$ steht, worin |
| $R^{20}$ | die oben angegebene Bedeutung von $R^{11}$ hat und mit dieser gleich oder verschieden ist, |
| A | für eine Bindung oder für einen Rest der Formel |

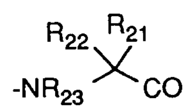

steht, worin

| | |
|---|---|
| $R^{21}$ und $R^{22}$ | gleich oder verschieden sind und die oben angegebene Bedeutung von $R^{13}$ und $R^{14}$ haben und mit dieser gleich oder verschieden sind, |
| $R^{23}$ | die oben angegebene Bedeutung von $R^2$, $R^3$ oder $R^5$ hat und mit dieser gleich oder verschieden ist, |
| $R^6$ | für Formyl oder Carboxy steht oder für geradkettiges oder verzweigtes Alkoxycarbonyl mit bis zu 4 Kohlenstoffatomen steht, oder für einen Rest der Formel $-CH_2-OR^{24}$ oder $-CH(OR^{25})_2$ steht, worin |
| $R^{24}$ und $R^{25}$ | gleich oder verschieden sind und Wasserstoff, geradkettiges oder verzweigtes Alkyl mit bis zu 3 Kohlenstoffatomen oder Benzyl bedeuten, |

und deren Salze,
mit der Maßgabe, daß A nicht für Valin stehen darf.

Außerdem wurden neue Verfahren zur Herstellung der erfindungsgemäßen Verbindungen der allgemeinen Formel (I) gefunden, dadurch gekennzeichnet, daß man Verbindungen der allgemeinen Formel (III)

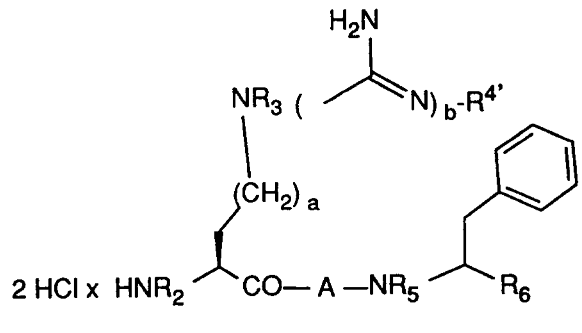

(III)

in welcher

A, a, b, $R^2$, $R^3$, $R^5$ und $R^6$ die oben angegebene Bedeutung haben,

und

$R^{4'}$ die oben angegebene Bedeutung von $R^4$ hat, aber nicht für Wasserstoff steht,

[A] im Fall, daß $R^1$ für den Rest der Formel $R^7$-$NR^8$-CO- steht, zunächst durch Umsetzung mit Verbindungen der allgemeinen Formel (IV)

$R^7$-N=C=O (IV)

in welcher

$R^7$ die oben angegebene Bedeutung hat,

in inerten Lösemitteln, in Anwesenheit einer Base, in die Verbindungen der allgemeinen Formel (V)

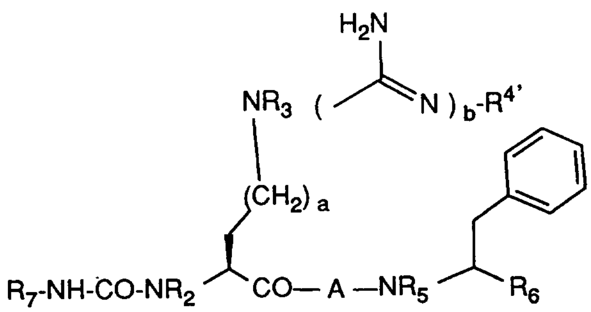

(V)

in welcher

A, a, b, $R^1$, $R^2$, $R^3$, $R^5$, $R^6$ und $R^7$ die oben angegebene Bedeutung haben,

überführt,

oder

[B] im Fall, daß $R^1 \neq R^7$-NH-CO- Verbindungen der allgemeinen Formel (III) mit Verbindungen der allgemeinen Formel (VI) oder (VII)

V-CO-W (VI) oder X-$SO_2$-$R^{11}$ (VII)

in welcher

$R^{11}$ die oben angegebene Bedeutung hat,

V den oben aufgeführten Bedeutungsumfang der Reste $R^9$-$(CH_2)_c$ oder $R^{10}$-$(CH_2)_d$-O- umfaßt

und

W und X gleich oder verschieden sind und Hydroxy oder einen typischen carbonsäureaktivierenden Rest, wie beispielsweise Chlor, bedeuten,

nach denen, in der Peptidchemie üblichen Methoden, in inerten organischen Lösemitteln und in Anwesenheit einer Base und eines Hilfsstoffes umsetzt,

und im Fall, daß $R^2$, $R^3$, $R^5$ und $R^8 \neq$ H gegebenenfalls eine Alkylierung nach üblichen Methoden anschließt,

und im Fall, $R^6$ = $CH_2$-OH, die Verbindugen der allgemeinen Formel (V) ($R^6$ = $COOCH_3$) nach üblichen Methoden, vorzugsweise aber mit Natriumborhydrid umsetzt,

und im Fall, $R^6$ = CHO, die Verbindungen der allgemeinen Formel (V), ausgehend von der Hydroxyme-

thylverbindung ($R^6$ = $CH_2$-OH) einer Oxidation unterzieht,
in Abhängigkeit des Restes $R^{4'}$, beispielsweise mit Fluorwasserstoffsäure oder Trifluoressigsäure zu $R^4$ = H umsetzt,
und im Fall einer Aminoschutzgruppe ($R^1$, $R^2$, $R^3$, $R^{4'}$ und $R^5$) diese nach denen in der Peptidchemie üblichen Methoden abspaltet,
und im Fall der Säuren die Ester verseift.

Das erfindungsgemäße Verfahren kann durch folgende Formelschema beispielhaft erläutert werden (Schema 1 - 3):

Schema 1:

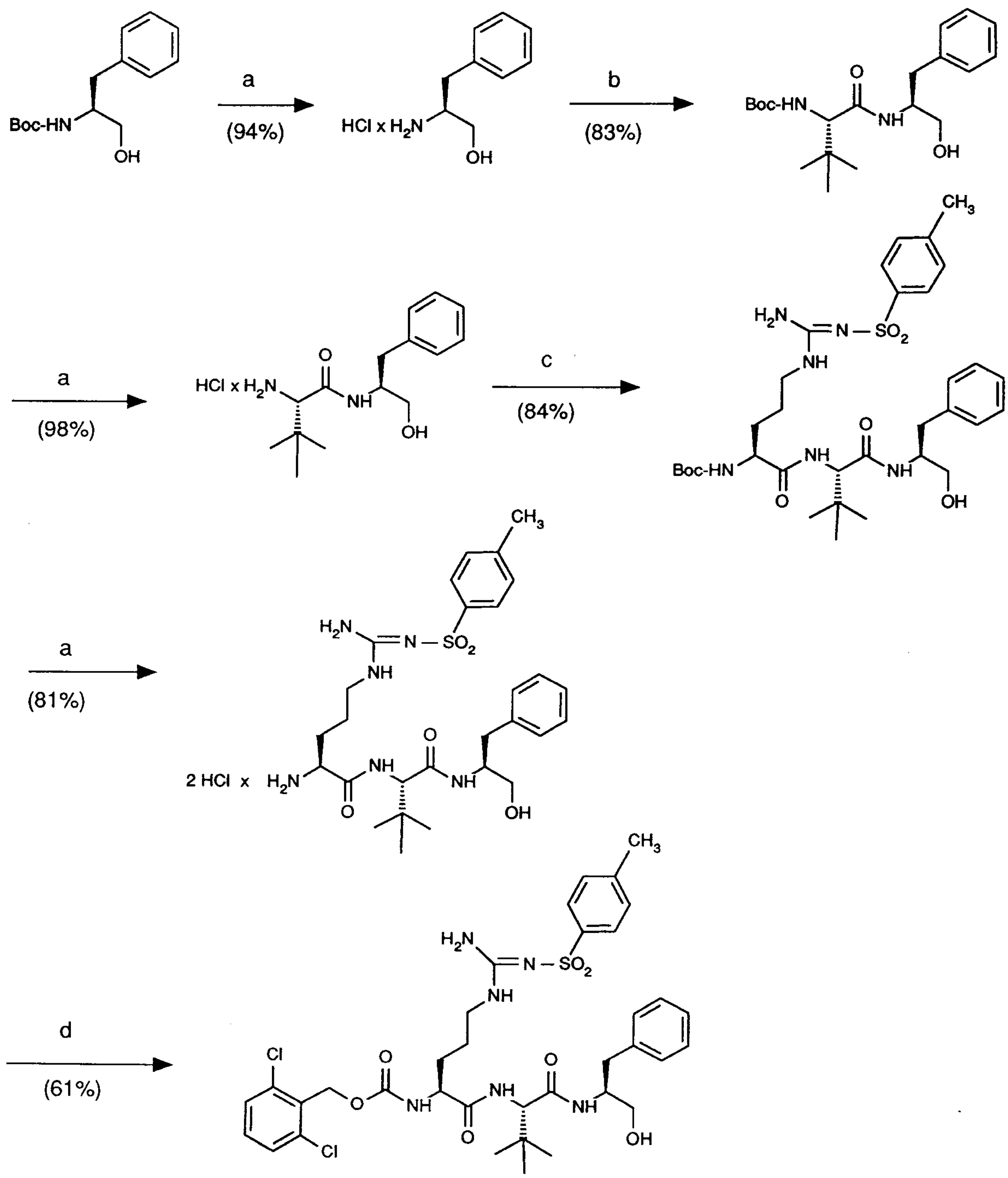

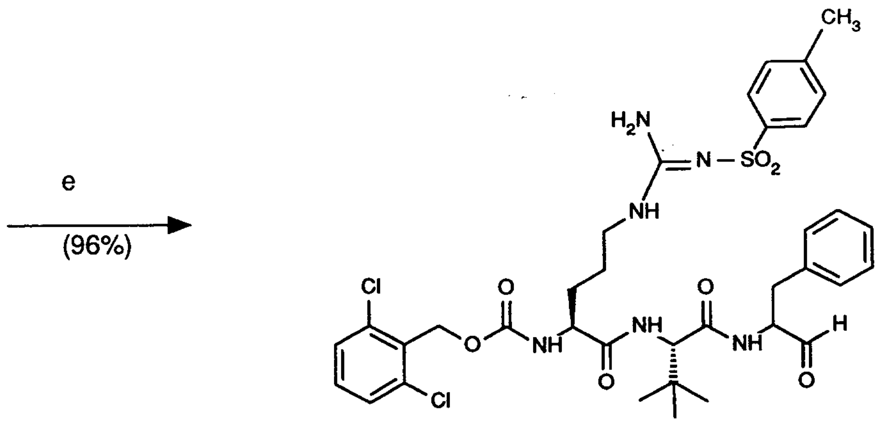


Reagenzien:

a) 4 N HCl in Dioxan; 30 min. Raumtemperatur
b) Boc-Gly(t-Bu)-OH, HOBT, DCC, $CH_2Cl_2$; 2 h Raumtemperatur
c) Boc-Arg (Tos)-OH, HOBT, DCC, $CH_2Cl_2$/DMF; 1 h Raumtemperatur
d) 2,6-Cl-$C_6H_3$-$CH_2OCOCl$, Dioxan, Wasser, pH 9-10, 2 h Raumtemperatur
e) Pyr$\times SO_3$, $NEt_3$, DMSO, 1 h Raumtemperatur

Schema 2:

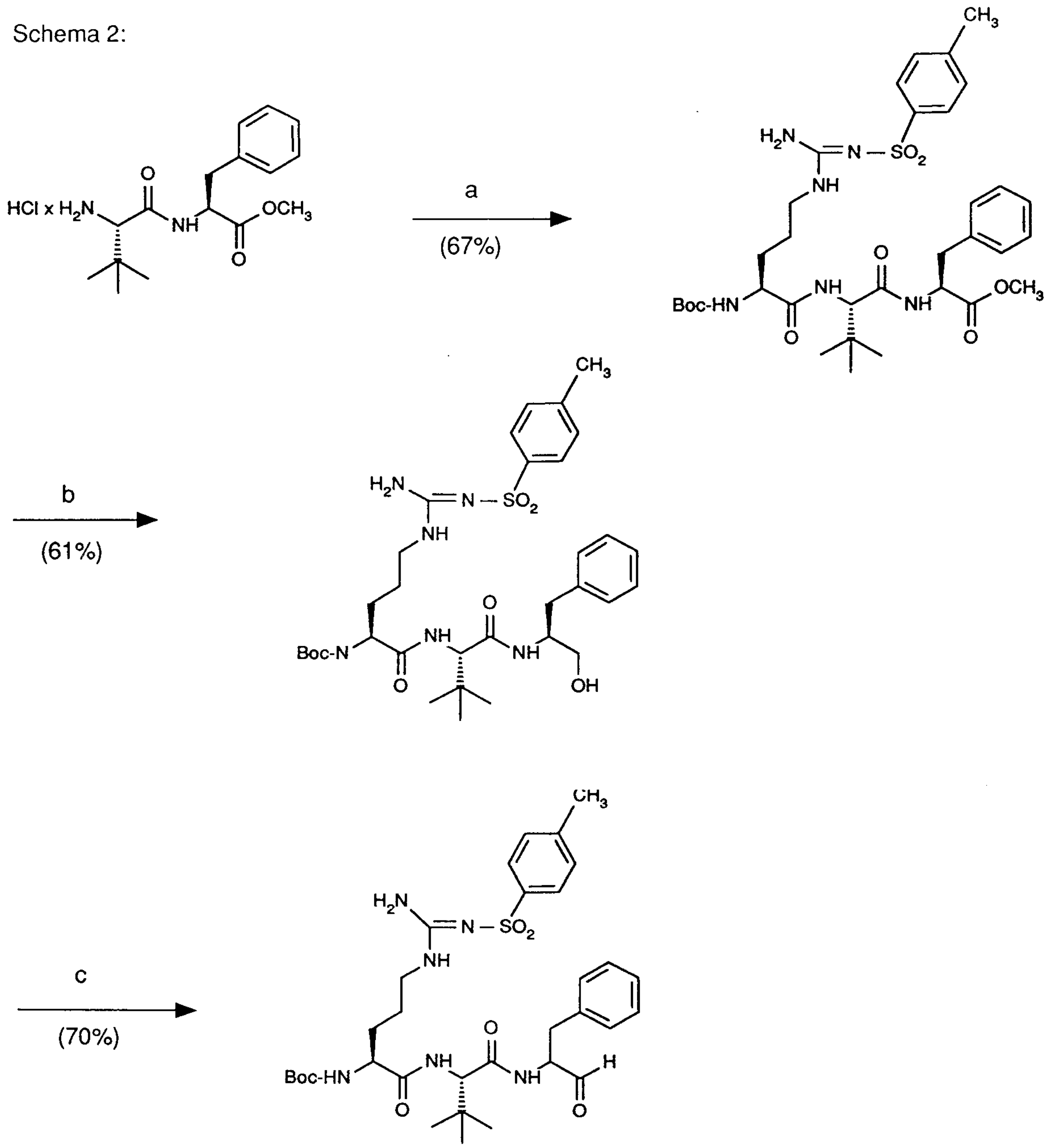


Reagentien:

a) Boc-Arg(Tos)-OH, HOBT, DCC, $CH_2Cl_2$, DMF, 1 h Raumtemperatur
b) $NaBH_4$, LiI, THF, MeOH, 5 h 40 °C
c) Pyr x $SO_3$, $NEt_3$, DMSO, 1 h Raumtemperatur

Schema 3:

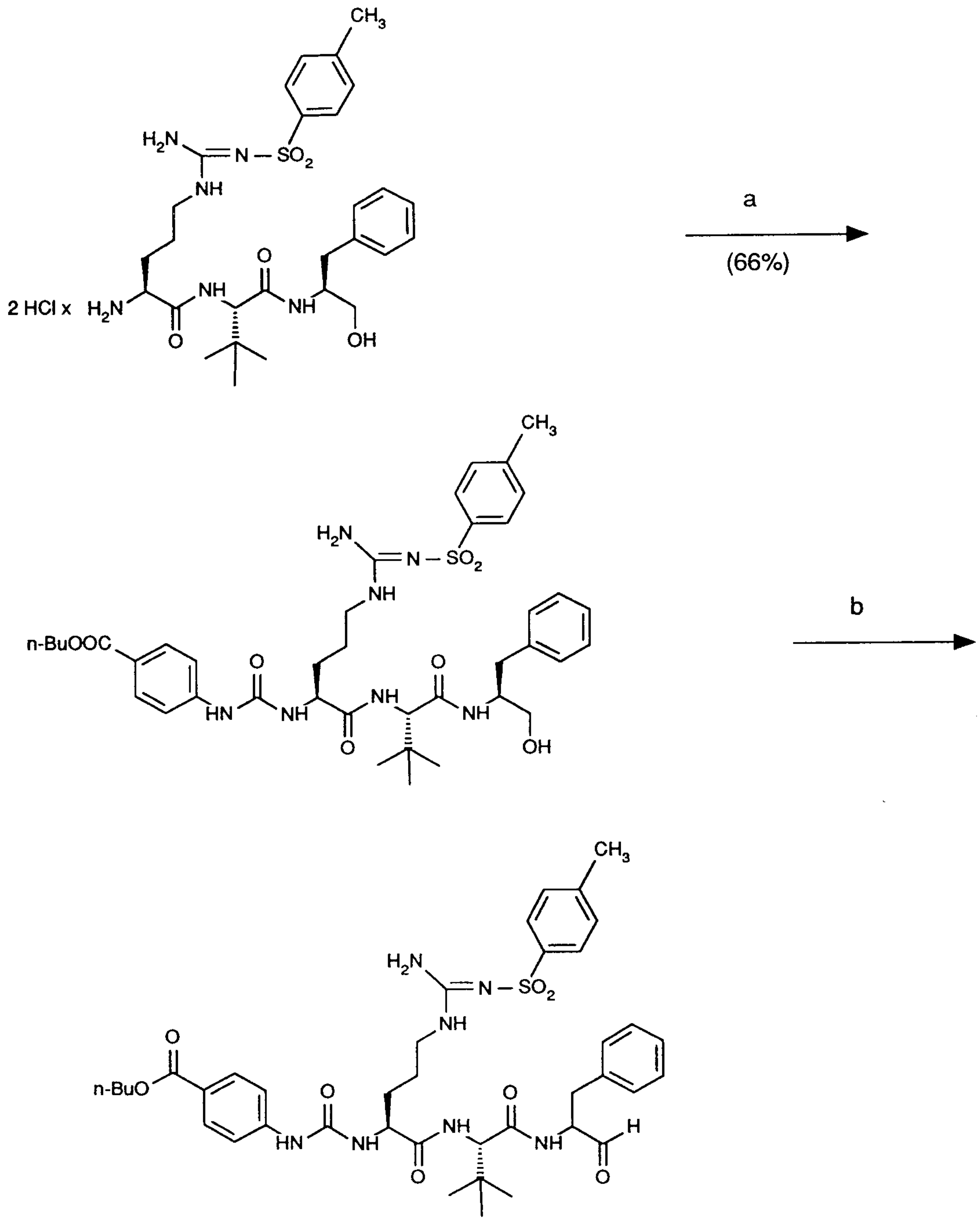


Reagentien:

a) n-BuOCO-$C_6H_4$-NCO, $NEt_3$, $CH_2Cl_2$; 30 min Raumtemperatur

b) Pyr x $SO_3$, $NEt_3$, DMSO, 1 h Raumtemperatur

Als Lösemittel eignen sich für alle Verfahrensschritte die üblichen inerten Lösemittel, die sich unter den Reaktionsbedindungen nicht verändern. Hierzu gehören bevorzugt organische Lösemittel wie Ether z.B. Diethylether, Glykolmono- oder -dimethylether, Dioxan oder Tetrahydrofuran, oder Kohlenwasserstoffe wie Benzol, p-Kresol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Losemittel, gegebenenfalls auch mit Wasser zu verwenden. Besonders bevorzugt sind Methylenchlorid, Tetrahydrofuran, Dioxan und Dioxan/Wasser.

Als Basen eignen sich organische Amine (Trialkyl($C_1$-$C_6$)amine wie beispielsweise Triethylamin oder Heterocyclen wie Pyridin, Methylpiperidin, Piperidin oder N-Methylmorpholin. Bevorzugt sind Triethylamin und N-Methylmorpholin.

Die Basen werden im allgemeinen in einer Menge von 0,1 mol bis 5 mol, bevorzugt von 1 mol bis 3 mol jeweils bezogen auf 1 mol der Verbindungen der allgemeinen Formel (III), (VI) und (VII) eingesetzt.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Reaktionen werden in einem Temperaturbereich von 0 °C bis 100 °C, vorzugsweise bei 0 °C bis 30 °C und bei Normaldruck durchgeführt.

Die Abspaltung der Aminoschutzgruppen erfolgt in an sich bekannter Weise.

Die Abspaltung der Tosylgruppe erfolgt im allgemeinen mit Fluorwasserstoffsäure (wasserfrei) in Gegenwart eines Scavengers, vorzugsweise p-Kresol oder mit Pyridiniumhydrofluorid [s. Matsuura et al., J.C.S. Chem. Comm. (1976), 451], in einem Temperaturbereich von -10 °C bis +30 °C, vorzugsweise bei 0 °C.

Als Hilfsstoffe für die jeweiligen Peptidkupplungen werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid, oder Carbonylverbindungen wie Carbonyldiimidazol, oder 1,2-Isoxazoliumverbindungen wie 2-Ethyl-5-phenyl-1-isoxazolium-3-sulfonat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri-(dimethylamino)-phosphoniurnhexafluorophosphat, oder 1-Hydroxybenzotriazol und als Basen Alkalicarbonate z.B. Natrium- oder Kaliumcarbonat, oder -hydrogencarbonat, oder organische Basen wie Trialkylamine z.B. Triethylamin, N-Ethylmorpholin, N-Methylpiperidin oder Diisopropylethylamin eingesetzt. Besonders bevorzugt sind Dicyclohexylcarbodiimid, N-Methylmorpholin und 1-Hydroxybenztriazol.

Die Verseifung der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Lithiumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat. Kaliumethanolat, Kaliummethanolat oder Kalium- tert.butanolat. Besonders bevorzugt werden Natriumhydroxid oder Lithiumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen. Bevorzugt ist Wasser/Tetrahydrofuran.

Die Verseifung wird im allgemeinen in einem Temperaturbereich von 0 °C bis +100 °C, bevorzugt von 0 °C bis +40 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base oder die Säure im allgemeinen in einer Menge von 1 bis 3 mol, bevorzugt von 1 bis 1,5 mol bezogen auf 1 mol des Esters eingesetzt. Besonders bevorzugt verwendet man molare Mengen der Reaktanden.

Bei der Durchführung der Reaktion entstehen im ersten Schritt die Salze der erfindungsgemäßen Verbindungen als Zwischenprodukte, die isoliert werden können. Die erfindungsgemäßen Säuren erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure, Zitronensäure oder Phosphorsäure. Es hat sich bei der Herstellung der Carbonsäuren als vorteilhaft erwiesen, die basische Reaktionsmischung der Verseifung in einem zweiten Schritt ohne Isolierung der Salze anzusäuern. Die Säuren können dann in üblicher Weise isoliert werden.

Die Reduktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden oder Boranen in inerten Lösemitteln, gegebenenfalls in Anwesenheit eines Katalysators durchgeführt werden.

Bevorzugt wird die Reduktion mit Hydriden, wie komplexen Borhydriden oder Aluminiumhydriden durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden. Bevorzugt sind Methanol und Tetrahydrofuran.

Als Katalysatoren können bei den Reduktionen auch Kalium- oder Lithiumjodid, bevorzugt Lithiumjodid eingesetzt werden.

Der Katalysator wird im allgemeinen in einer Menge von 0,1 mol bis 5 mol, bevorzugt von 1 mol bis 3 mol jeweils bezogen auf 1 mol des zu reduzierenden Esters eingesetzt.

Die Umsetzung kann bei normalem, erhöhtem oder bei erniedrigtem Druck durchgeführt werden (z.B. 0,5 bis 5 bar). Im allgemeinen arbeitet man bei Normaldruck.

Die Reduktionen werden im allgemeinen in einem Temperaturbereich von 0 °C bis +60 °C, vorzugsweise bei +10 °C bis +40 °C durchgeführt.

Die Oxidation von Alkoholgruppen zu den entsprechenden Aldehyden erfolgt im allgemeinen in einem der oben aufgeführten Lösemitteln, in Anwesenheit einer der oben aufgeführten Basen mit Oxidationsmitteln, wie beispielsweise Kaliumpermanganat, Brom, Jones-Reagenz, Pyridinium-dichromat, Pyridinium-chlorochromat, Pyridin-Schwefeltrioxid-Komplex oder mit Chlorlauge und 2,2,6,6-Tetramethylpiperidin-1-oxyl (TEMPO) [Org. Synth. 69, 212 (1990)] oder Oxalylchlorid [Swern-Oxidation (ClCOCOCl / DMSO / $CH_2Cl_2$ / $NEt_3$) z.B. nach R.E. Ireland et al., J. Org. Chem. 50, 2199 (1985)]. Bevorzugt erfolgt die Oxidation mit Pyridin-Schwefeltrioxid-Komplex in Dimethylsulfoxid in Anwesenheit von Triethylamin.

Die Oxidation erfolgt im allgemeinen in einem Temperaturbereich von 0 °C bis +50 °C, vorzugsweise bei Raumtemperatur und Normaldruck.

Die Alkylierung wird in den oben aufgeführten Lösemitteln bei Temperaturen von 0 °C bis +150 °C, vorzugsweise bei +20 °C bis +100 °C bei Normaldruck durchgeführt.

Als Lösemittel für die Alkylierung eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dimethylformamid. Bei der Alkylierung kann auch Natriumhydrid als Base eingesetzt werden.

Die Verbindungen der allgemeinen Formel (III) sind größtenteils neu und können dann nach denen in der Peptid-Chemie üblichen Methoden hergestellt werden, indem man beispielsweise Verbindungen der allgemeinen Formel (VIII)

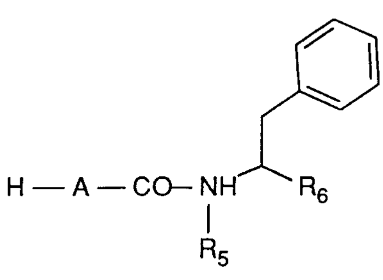

(VIII)

in welcher

$R^5$, $R^6$ und A die oben angegebene Bedeutung haben,

mit den Aminosäurederivaten der Formel (IX)

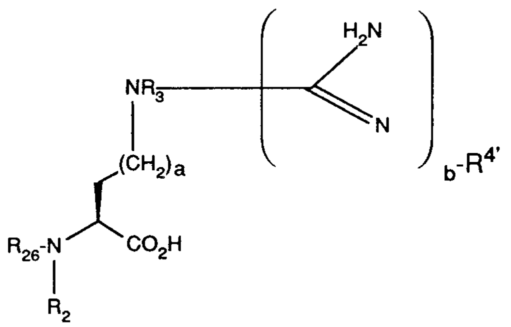 (IX)

in welcher

a, b, $R^2$, $R^3$ und $R^{4'}$ die oben angegebene Bedeutung haben

und

$R^{26}$ für eine der oben aufgeführten Aminoschutzgruppen, vorzugsweise 9-Fluorenylmethoxycarbonyl (Fmoc), tert.Butoxycarbonyl (Boc) oder Benzyloxycarbonyl (Z) steht,

in einem der oben angegebenen Lösemittel, vorzugsweise Methylenchlorid, in Anwesenheit eines Hilfsstoffes und/oder Base, vorzugsweise HOBT und Dicyclohexylcarbodiimid, umsetzt und anschließend, ebenfalls nach üblichen Methoden, die Aminoschutzgruppe abspaltet und zwar vorzugsweise Boc mit Salzsäure in Dioxan, Fmoc mit Piperidin und Z mit HBr/HOAc oder durch Hydrogenolyse.

Alle Verfahrensschritte erfolgen bei Normaldruck und in einem Temperaturbereich von 0°C bis Raumtemperatur, vorzugsweise bei Raumtemperatur.

Die Verbindungen der allgemeinen Formeln (VIII) und (IX) sind größtenteils bekannt oder können nach üblichen Methoden hergestellt werden [vgl. J. Chem. Res., Synop., (2), 62-63 ; DE 36 04 510]..

Die Verbindungen der allgemeinen Formel (V) sind ebenfalls bekannt [vgl. US 4 929 736].

Die Verbindungen der allgemeinen Formeln (VI) und (VII) sind bekannt.

Die Verbindungen zeigen eine antivirale Wirkung gegenüber Retroviren und Vertretern der Gruppe der Herpetoviridae, besonders gegenüber dem humanen Cytomegalovirus (HCMV).

Die Anti-HCMV-Wirkung wurde in einem Screening-Testsystem in 96-Well-Mikrotiterplatten unter Zuhilfenahme von humanen embryonalen Lungenfibroblasten (HELF)-Zellkulturen bestimmt. Der Einfluß der Substanzen auf die Ausbreitung des cytopathogenen Effektes wurde im Vergleich zu der Referenzsubstanz Ganciclovir ($Cymevene^R$-Natrium), einem klinisch zugelassenen anti-HCMV-Chemotherapeutikum, bestimmt.

Die in DMSO (Dimethylsulfoxid) gelösten Substanzen (50 mM) werden auf Mikrotiterplatten (96-Well) in Endkonzentrationen von 1000 - 0,00048 µM (micromolar) in Doppelbestimmungen (4 Substanzen/Platte) untersucht. Toxische und cytostatische Substanzwirkungen werden dabei miterfaßt. Nach den entsprechenden Substanzverdünnungen (1:2) auf der Mikrotiterplatte wird eine Suspension von 50 - 100 HCMV-infizierten HELF-Zellen und 3 x $10^4$ nichtinfizierten HELF-Zellen in Eagle's MEM (Minimal Essential Medium) mit 10% fötalem Kälberserum in jedes Näpfchen gegeben, und die Platten bei 37°C in einem $CO_2$-Brutschrank über 6 Tage inkubiert. Nach dieser Zeit ist der Zellrasen in den substanzfreien Viruskontrollen, ausgehend von 50 - 100 infektiösen Zentren, durch den cytopathogenen Effekt (CPE) des HCMV völlig zerstört (100% CPE). Nach einer Anfärbung mit Neutralrot und Fixierung mit Formaldehyd / Methanol werden die Platten mit Hilfe eines Projektions-Mikroskopes (Plaque-Viewer) ausgewertet. Die Ergebnisse sind für einige Verbindungen in der folgenden Tabelle zusammengefaßt:

Tabelle: Anti-HCMV (Davis)-Aktivität und antizelluläre Wirkung

| Bsp.-Nr. | $CIC_{50}$ (µM) 1) (HELF) | $IC_{50}$ (µM) 2) (HCMV) | SI 3) |
|---|---|---|---|
| 22 | 62,5 | 1,95 | 32 |
| 50 | 2,8 | 0,004 | 700 |
| 52 | 15,6 | 0,125 | 125 |
| 62 | 14,3 | 0,163 | 88 |
| 63 | 93,8 | 0,49 | 191 |
| 67 | 23,4 | 0,214 | 109 |
| 68 | 344 | 4,9 | 70 |
| 69 | 2,6 | 0,013 | 200 |
| 70 | 51 | 0,98 | 52 |
| 71 | 187,5 | 0,98 | 191 |
| 72 | 3,44 | 0,023 | 150 |
| 73 | 0,56 | 0,0013 | 430 |
| 74 | 7,8 | 0,046 | 170 |
| 75 | 0,86 | <0,0005 | 1720 |
| 77 | 9,8 | 0,061 | 160 |
| 83 | 62,5 | 0,244 | 256 |
| Cymevene$^R$-Na | 125 | 2-4 | 32-64 |

1) $CIC_{50}$ = Höchste Konzentration, die keine offensichtliche antizelluläre Wirkung zeigt.

2) $IC_{50}$ = Konzentration der erfindungsgemäßen Verbindung, die eine 50%ige Hemmung des CPE bewirkt.

3) $SI = \frac{CIC_{50}}{IC_{50}}$ = Selektivitätsindex

Es wurde nun gefunden, daß die erfindungsgemäßen Verbindungen, die Vermehrung des HCMV in HELF-Zellen in z.T. 10-50fach niedrigeren Konzentrationen als Cymeven$^R$-Natrium hemmen und einen mehrfach höheren Selektivitätsindex aufweisen.

Die erfindungsgemäßen Verbindungen stellen somit wertvolle Wirkstoffe zur Behandlung und Prophylaxe von Erkrankungen durch das humane Cytomegalievirus dar. Als Indikationsgebiete können beispielsweise genannt werden:

1) Behandlung und Prophylaxe von Cytomegalievirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.

2) Behandlung und Prophylaxe von HCMV-Infektionen bei AIDS-Patienten (Retinitis, Pneumonitis, gastrointestinale Infektionen).

3) Behandlung und Prophylaxe von HCMV-Infektionen bei Schwangeren, Neugeborenen und Kleinkindern.

Darüberhinaus wurde überraschend gefunden, daß die Verbindungen der allgemeinen Formel (I) eine Wirkung gegen Retroviren besitzen. Dies wird mit einem HIV-spezifischen Protease-Enzymtest belegt.
Die Ergebnisse der unten aufgeführten Beispiele wurden nach dem in den folgenden Literaturangabe [vgl. Hansen, J., Billich, S., Schulze, T., Sukrow, S. and Mölling, K. (1988), EMBO Journal, Vol, 7, No. 6, pp. 1785 - 1791] beschriebenen HIV-Testsystem ermittelt: Gereinigte HIV-Protease wurde mit synthetischem

Peptid, das eine Schnittstelle im Gag-Precursor-Protein imitiert und eine in vivo-Spaltstelle der HIV-Protease darstellt, inkubiert. Die entstandenen Spaltprodukte des synthetischen Peptids wurden über Reverse Phase High Performance Liquid Chromatography (RP-HPLC) analysiert. Die angegebenen $IC_{50}$-Werte beziehen sich auf die Substanzkonzentration, die unter den oben aufgeführten Testbedingungen eine 50%ige Hemmung der Protease-Aktivität bewirkt.

Tabelle

| $IC_{50}$ (RP-HPLC) (μM) | |
|---|---|
| Bsp.-Nr. | HIV-1 |
| 43 | 2,6 |
| 47 | 13 |
| 52 | 1,9 |
| 71 | 0,21 |
| 80 | 19 |

Der neue Wirkstoff kann in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral, parenteral oder topisch, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 25 mg/kg, vorzugsweise 0,1 bis 10 mg/kg Köpergewicht.

Trotzdem kann es gegebenenfalls erforderlich sein, von den genannten Mengen abzuweichen, und zwar in Abhängigkeit vom Körpergewicht bzw. der Art des Appllkationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese in mehreren Einzelgaben über den Tag zu verteilen.

Als Enzyminhibitoren sind die erfindungsgemäßen Verbindungen einsetzbar in allen Bereichen die allgemein für Inhibitoren bekannt sind. Gemeint dabei ist zum Beispiel der Einsatz als Affinitätslabel für die Affinitätschromatographie bei der Reinigung von Proteasen. Sie können aber auch als Hilfsmittel dienen für die Aufklärung von Reaktionsmechanismen der Enzyme sowie zur Verbesserung der Spezifität von diagnostischen Verfahren.

Anhang zum experimentellen Teil

I. Aminosäuren

Im allgemeinen erfolgt die Bezeichnung der Konfiguration durch das Vorausstellen eines L bzw. D vor der Aminosäureabkürzung, im Fall des Racemats durch ein D,L-, wobei zur Vereinfachung bei L-Aminosäuren die Konfigurationsbezeichnung unterbleiben kann und dann nur im Fall der D-Form bzw. des D,L-Gemisches eine explizierte Bezeichnung erfolgt.

Ala L-Alanin

| | |
|---|---|
| Arg | L-Arginin |
| Ile | L-Isoleucin |
| Leu | L-Leucin |
| Phe | L-Phenylalanin |
| Gly | Glycin |
| Orn | L-Ornithin |
| Lys | L-Lysin |

-Gly(t-Bu)-

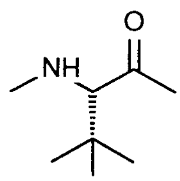

-$NCH_3$-Ile-

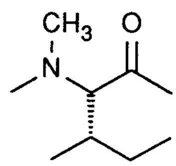

-$NCH_3$-Ala-

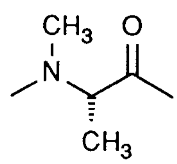

-$NCH_3$-Gly-

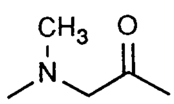

-ßAla-

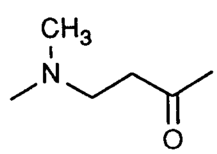

-Aib-

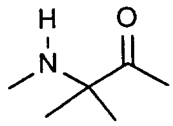

-Arg(Tos)-

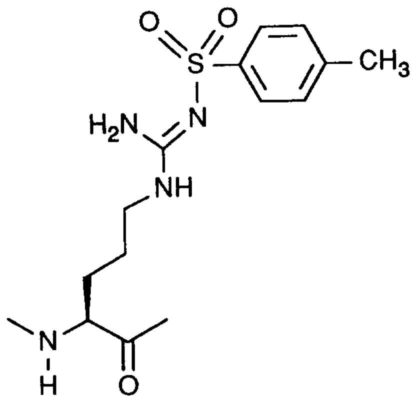

-Arg($NO_2$)-

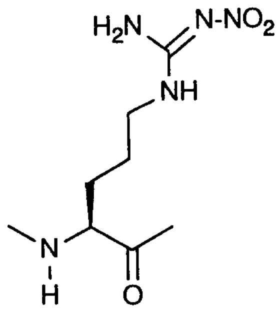

-Lys(Tos)-

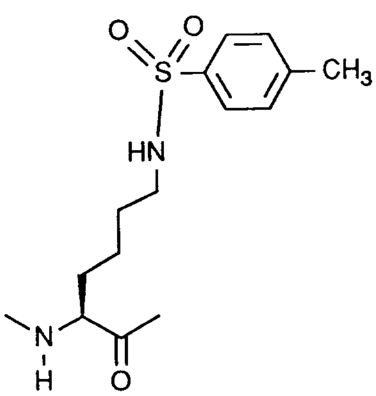

-Orn(Z)-

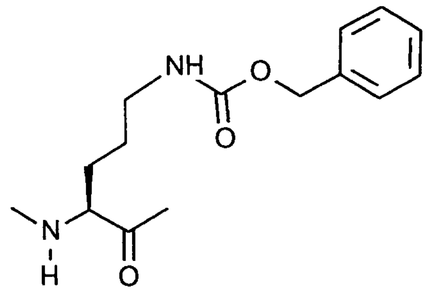

II. Abkürzungen

| | |
|---|---|
| Z | Benzyloxycarbonyl |
| Boc | tert.Butyloxycarbonyl |
| CMCT | 1-Cyclohexyl-3-(2-morpholino-ethyl)-carbodiimidmetho-p-toluolsulfonat |
| DCC | Dicyclohexylcarbodiimid |
| DMF | Dimethylformamid |
| HOBT | 1-Hydroxybenzotriazol |
| Ph | Phenyl |
| THF | Tetrahydrofuran |
| DMSO | Dimethylsulfoxid |
| Fmoc | 9-Fluorenylmethoxycarbonyl |

III. Liste der verwendeten Laufmittelgemische zur Chromatographie

I: Dichlormethan:Methanol
II: Toluol:Ethylacetat
III: Acetonitril:Wasser

Ausgangsverbindungen

Beispiel I

(2S)-2-Amino-3-phenyl-propan-1-ol Hydrochlorid

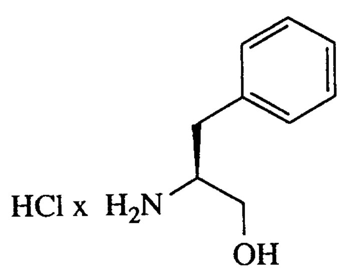

Eine Lösung von 20,10 g (80,00 mmol) (S)-2-(tert.Butoxycarbonylamino-1-phenyl-propan-1-ol [J. Med. Chem. 33, 2707 (1990)] in 200 ml einer 4 N Lösung gasförmigen Chlorwasserstoffs in wasserfreiem Dioxan wird 30 min bei Raumtemperatur gerührt. Danach gibt man 60 ml Toloul zu und engt im Vakuum ein. Dieser Vorgang wird noch zweimal wiederholt, dann wird der Rückstand mit wenig Ether verrieben, abgesaugt und im Hochvakuum über KOH getrocknet. Man erhält 14,14 g (94% der Theorie) der Titelverbindung als farblose Kristalle.
Schmp.: 148-150 °C (Ether)
$R_f$ = 0,25 (Acetonitril : Wasser 9:1)
MS (DCI, $NH_3$) m/z = 152 $(M+H)^+$
IR (KBr) 3357, 2928, 1571, 1495, 1456, 1026, 738, 708 $cm^{-1}$

$[\alpha]^{20}_D$ = -4,2° (c = 2,94, $CH_3OH$)
$^1$H-NMR (300 MHz, $CD_3OD$) $\delta$ = 2,95 (d, 2H, J = 7,5 Hz, $CH_2$); 3,50 (m, 2H); 3,70 (m, 1H); 7,30 (m, 5H, Ph).
$C_9H_{13}NO$ x HCl (187,67)

Beispiel II

(2S)-2-[N-(tert.Butoxycarbonyl)-S-(tert.butyl)glycinyl]amino-3-phenyl-propan-1-ol

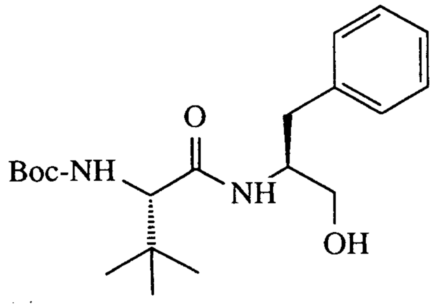

Eine auf 0°C gekühlte, gerührte Lösung von 25,44 g (110,00 mmol) N-(tert.Butoxycarbonyl)-S-(tert.butyl)-glycin und 16,85 g (110,00 mmol) HOBT in 300 ml wasserfreiem Dichlormethan wird mit 21,70 g (105,00 mmol) DCC versetzt und 5 min gerührt. Danach wird eine Lösung von 18,80 g (100,00 mmol) der Verbindung aus Beispiel I und 38.50 ml (350,00 mmol) N-Methylmorpholin in 300 ml Dichlormethan zugetropft. Das Kühlbad wird entfernt und die Reaktionsmischung darf 12 h bei Raumtemperatur rühren. Das Ende der Reaktion wird dünnschichtchromatographisch festgestellt. Man trennt den entstandenen Harnstoff durch Filtration ab, engt das Filtrat im Vakuum ein und reinigt das Rohprodukt durch Chromatographie an 450 g Kieselgel (Dichlormethan: Methanol 95:5). Man erhält 30,23 g (83% der Theorie) der Titelverbindung als farblose Kristalle.
Schmp.: 188°C
$R_f$ = 0,23, I (95:5)
MS (FAB): m/z = $(M+H)^+$ 365
Wie für Beispiel II beschrieben erhielt man durch Kondensation der Verbindung aus Beispiel I mit den entsprechenden N-geschützten Aminosäuren die in Tabelle I aufgeführten Verbindungen:

Tabelle I:

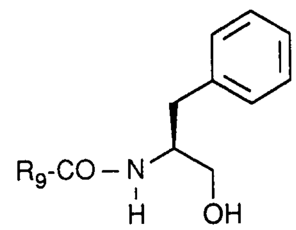

| Bsp.-Nr. | $R^9$ | Ausbeute (% d.Th.) | MS (FAB) m/z $(M+H)^+$ | $R_f$ / Laufmittel-verhältnis | Schmp. (°C) |
|---|---|---|---|---|---|
| III | 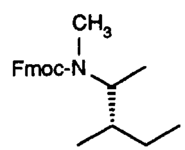 | 57 | 501 | 0,31, I (95:5) | Öl |
| IV | 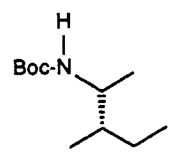 | 83 | 365 | 0,50, I (9:1) | 133 |
| V | 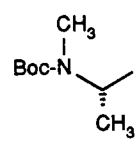 | 97 | 337 | 0,42, I (9:1) | 98 |

Fortsetzung Tabelle I:

| Bsp.-Nr. | $R^9$ | Ausbeute (% d.Th.) | MS (FAB) m/z $(M+H)^+$ | $R_f$ / Laufmittel-verhältnis | Schmp. (°C) |
| --- | --- | --- | --- | --- | --- |
| VI | 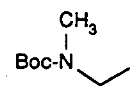 | 86 | 323 | 0,40, I (9:1) | Schaum |
| VII | 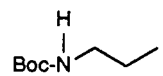 | 87 | 323 | 0,31, I (9:1) | amorph |
| VIII | 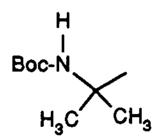 | 86 | 337 | 0,21, I (9:1) | 79 |

Beispiel IX

(2S)-2-[S-(tert.Butyl)glycinyl]amino-3-phenyl-propan-1-ol

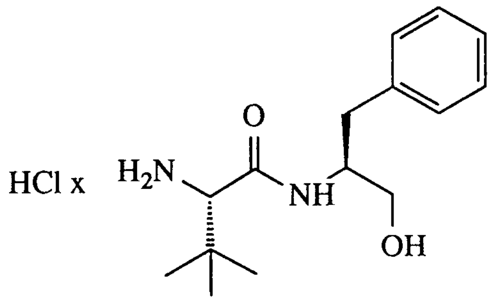

Eine Lösung von 12,90 g (35,40 mmol) der Verbindung aus Beispiel II in 60 ml wasserfreien Dioxan wird mit 60 ml einer 4 N Lösung gasförmigen Chlorwasserstoffs in wasserfreiem Dioxan versetzt und 30 min bei Raumtemperatur gerührt. Danach gibt man 60 ml Toluol zu und engt im Vakuum ein. Dieser Vorgang wird noch zweimal wiederholt, dann wird der Rückstand mit 200 ml Ether verrieben, abgesaugt und im Hochvakuum über KOH getrocknet. Man erhält 10,45 g (98% der Theorie) der Titelverbindung als farblose Kristalle.
Schmp.: ab 105 °C (Zers.)
$R_f$ = 0,20, III (95:5)
MS (FAB) m/z = 265 $(M+H)^+$
Wie für Beispiel IX beschrieben erhält man nach Abspaltung der Aminoschutzgruppen aus den in Tabelle I beschriebenen Verbindungen die in Tabelle II aufgeführten Hydrochloride:

Tabelle II:

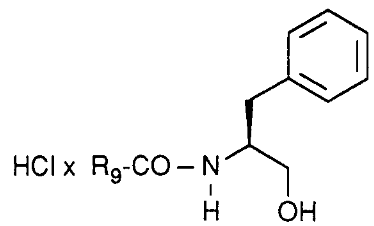

| Bsp.-Nr. | $R^9$ | Ausbeute (% d.Th.) | MS (FAB) m/z $(M+H)^+$ | $R_f$ / Laufmittel-verhältnis | Schmp. (°C) |
|---|---|---|---|---|---|
| X | 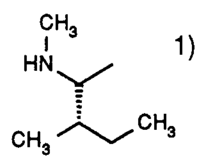 1) | 77 | 279 | 0,19, I (9:1) | 90 |
| XI | 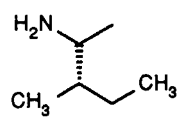 | 98 | 265 | 0,43 , III (9:1) | 126 |
| XII | 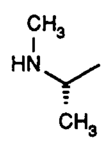 | 87 | 237 | 0,05, I (85:5) | 164 |

Fortsetzung Tabelle II:

| Bsp.-Nr. | $R^9$ | Ausbeute (% d.Th.) | MS (FAB) m/z $(M+H)^+$ | $R_f$ / Laufmittel-verhältnis | Schmp. (°C) |
|---|---|---|---|---|---|
| XIII | 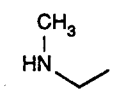 | 96 | 223 | 0,05, I (85:15) | 177 |
| XIV | $H_2N-CH_2-CH_2-$ | 92 | 223 | 0,05, I (85:15) | 127 |
| XV | 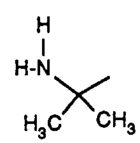 | 78 | 237 | 0,10, I (8:2) | 107 |

1) freie Base, erhalten durch Abspaltung der Fmoc-Gruppe mit Piperidin

Herstellungsbeispiele

Beispiel 1

(2S)-2-[Nα-(tert.Butoxycarbonyl)-$N^G$-(4-methyl-phenylsulfonyl)-S-arginyl-S-tert.-butyl-glycinyl]amino-3-phenyl-propan-1-ol

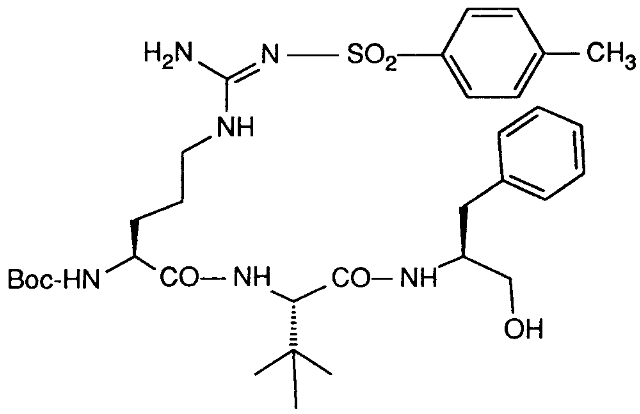

Methode A:

Eine auf 0°C gekühlte, gerührte Lösung von 15,67 g (36,56 mmol) $N_\alpha$-(tert.Butoxycarbonyl)-$N^G$-(4-methylphenylsulfonyl)-S-arginin und 6,16 g (40,22 mmol) HOBT in 120 ml wasserfreiem Dichlormethan und 12 ml DMF wird mit 7,92 g (38,39 mmol) DCC versetzt und 5 min gerührt. Danach wird eine Lösung von 10,00 g (33,24 mmol) der Verbindung aus Beispiel IX und 14,60 ml (133,00 mmol) N-Methylmorpholin in 70 ml Dichlormethan und 7 ml DMF zugetropft. Das Kühlbad wird entfernt und die Reaktionsmischung darf 1 h bei Raumtemperatur rühren. Das Ende der Reaktion wird dünnschichtchromatographisch festgestellt. Man trennt den entstandenen Harnstoff durch Filtration ab, engt das Filtrat im Vakuum ein und reinigt das Rohprodukt durch Chromatographie an 500 g Kieselgel (Dichlormethan : Methanol 9:1). Man erhält 18,95 g (84% der Theorie) der Titelverbindung als farblosen Schaum.

MS (FAB): m/z $(M+H)^+$ = 675

$R_f$ = 0,14 (Dichlormethan: Methanol 9:1)

Methode B:

Zu einer gerührten, auf 40°C geheizten Lösung von 1,36 g (36,00 mmol) Natriumborhydrid und 4,83 g (36,00 mmol) Lithiumjodid in 90 ml THF gibt man innerhalb von 10 min portionsweise 16,87 g (24,00 mmol) der Verbindung aus Beispiel 3. Zu diesem Gemisch tropft man innerhalb von 5 h bei 40°C langsam 25 ml Methanol. Das Ende der Reaktion wird dünnschichtchromatographisch festgestellt und die Reaktionsmischung wird in 250 ml einer verdünnten Lösung von Citronensäure geschüttet. Das Gemisch wird mit 4 mal 50 ml Ethylacetat extrahiert und die vereinigten Extrakte werden über $MgSO_4$ getrocknet. Nach Abdampfen des Lösemittels im Vakuum und Chromatographie des Rückstands an 450 g Kieselgel (Dichlormethan: Methanol 9:1) erhält man 9,90 g (61%) der Titelverbindung.

Beispiel 2

(2S)-2-[$N^G$-(4-Methyl-phenylsulfonyl)-S-arginyl-S-tert.butyl-glycinyl]amino-3-phenyl-propan-1-ol Dihydrochlorid

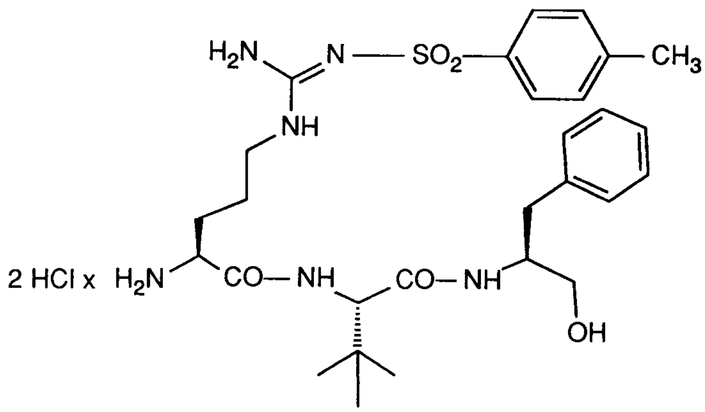

Wie für Beispiel I beschrieben erhält man aus 18,50 g (27,41 mmol) der Verbindung aus Beispiel 1 13,51 g (81% der Theorie) der Titelverbindung als farbloses Pulver.
Schmp.: 186 °C (Zers.)
$R_f$ = 0,34 (Dichlormethan: Methanol 85:15)
MS (FAB) m/z = 575 $(M+H)^+$

Beispiel 3

Nα-(tert.Butoxycarbonyl)-$N^G$-(4-methyl-phenylsulfonyl)-S-arginyl-S-tert.-butyl-glycinyl-S-phenylalanin-methylester

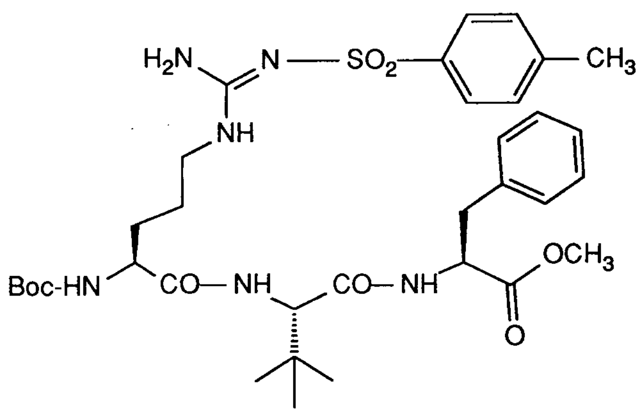

Wie für Beispiel 1 (Methode A) beschrieben erhält man aus 4,71 g (11,00 mmol) $N_\alpha$-(tert.Butoxycarbonyl)-$N^G$-(4-methyl-phenylsulfonyl)-S-arginin und 3,15 g (9,57 mmol) S-(tert.Butyl)glycin-S-phenylalanin-methylester Hydrochlorid nach 4 h bei Raumtemperatur 4,55 g (67% der Theorie) der Titelverbindung als amorphes Pulver.
$R_f$ = 0,35 (Ethylacetat)
MS (FAB) m/z = 703 $(M+H)^+$

Beispiel 4

$N^G$-(4-Methyl-phenylsulfonyl)-S-arginyl-S-tert.butyl-glycinyl-S-phenylalanin-methylester Dihydrochlorid

2 HCl x 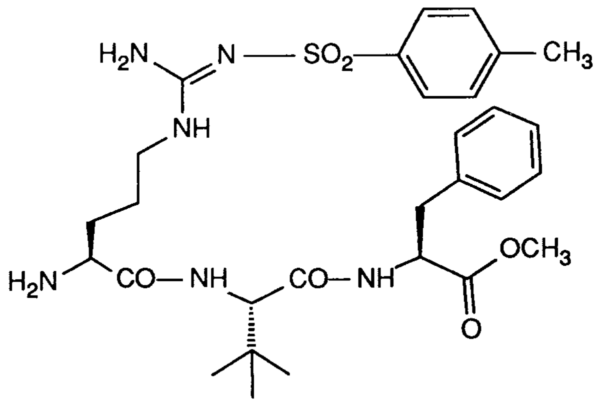

Wie für Beispiel I beschrieben erhält man aus 3,85 g (5,50 mmol) der Verbindung aus Beispiel 3 3,18 g (86% der Theorie) der Titelverbindung als farbloses Pulver.
Schmp.: ab 163 ° C (Zers.)
$R_f$ = 0,22 (Dichlormethan: Methanol 9:1)

Beispiel 5

Nα-(tert.Butoxycarbonyl)-$N^G$-(4-methyl-phenyl-sulfonyl)-S-arginyl-S-tert.-butyl-glycinyl-S-phenylalanin

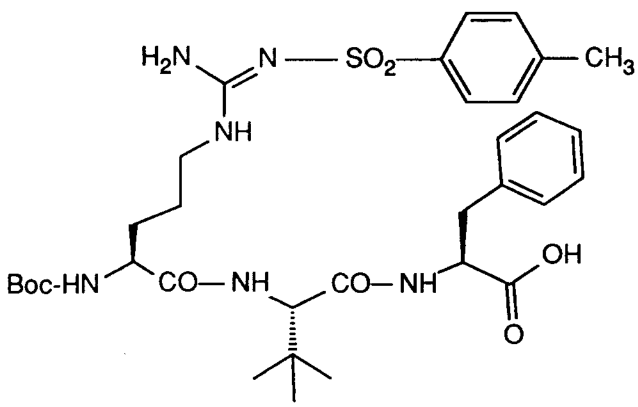

Eine Lösung von 703 mg (1,00 mmol) der Verbindung aus Beispiel 3 in 6 ml THF und 8 ml Wasser wird mit 91 mg (2,00 mmol) Lithiumhydroxid-hydrat versetzt und 30 min bei Raumtemperatur gerührt. Danach wird die Reaktionsmischung in 40 ml Ethylacetat geschüttet. Die organische Phase wird abgetrennt und die wäßrige Phase erneut mit 10 ml Ethylacetat extrahiert. Die wäßrige Phase wird am Rotationsverdampfer von Lösemittelresten befreit und mit 0,5 N Salzsäure auf pH 5,2 gestellt. Der entstandene Niederschlag wird 10 min gut durchgerührt, durch Filtration abgetrennt und im Hochvakuum zunächst über KOH, dann über Sicapent getrocknet. Man erhält 412 mg (60%) der Titelverbindung als amorphes Pulver.
Schmp.: 72 ° C (Zers.)
$R_f$ = 0,23 (Acetonitril: Wasser = 9:1)
MS (FAB) m/z = 689 $(M+H)^+$
Wie für Beispiel 1 beschrieben erhält man durch Kondensation der Amine aus Tabelle II mit verschiedenen geschützten Aminosäuren die in Tabelle 1 beschriebenen Verbindungen:

Tabelle 1:

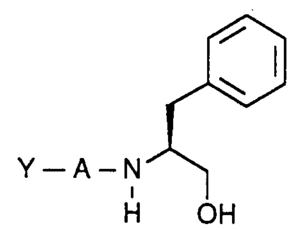

| Bsp.-Nr. | Y | A | Ausbeute (% d.Th.) | MS (FAB) m/z $(M+H)^+$ | $R_f$ / Laufmittel-verhältnis | Schmp. (°C) |
|---|---|---|---|---|---|---|
| 6 | Boc-Arg(Tos)- | —— | 97 | 562 | 0,09, I (9:1) | Schaum |
| 7 | Boc-Arg(Tos)- | -Gly(t-Bu)- | 84 | 675 | 0,14, I (9:1) | amorph |
| 8 | Z-Arg(Tos)- | -Gly(t-Bu)- | 64 | 709 | 0,29, I (9:1) | 110 |
| 9 | Boc-Arg(Tos)- | -ß-Ala- | 50 | 633 | 0,31, I (9:1) | 102 |
| 10 | Boc-Arg(Tos)- | -Ile- | 70 | 675 | 0,24, I (9:1) | 113 |

Beispiel 11

(2S)-2-[$N_\alpha$-(tert.Butoxycarbonyl)-$N^G$-(4-methyl-phenylsulfonyl)-S-arginyl-(S)-(N-methyl)-isoleucinyl]amino-S-phenyl-propan-1-ol

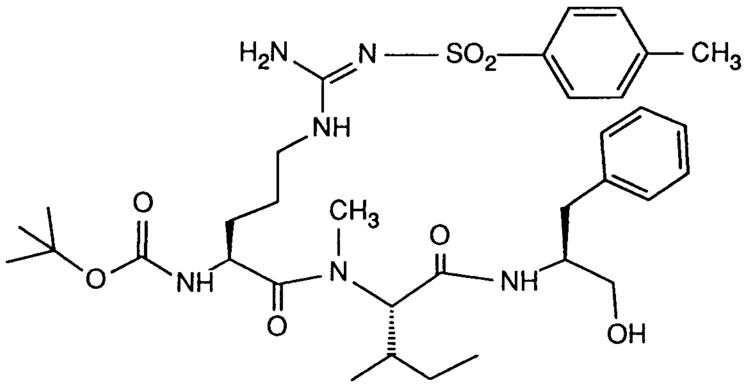

Eine gerührte, auf -10 ° C gekühlte Suspension von 600 mg (2,16 mmol) der Verbindung aus Beispiel X und 926 mg (2,16 mmol) $N_\alpha$-(tert.Butoxycarbonyl)-$N^G$-(4-methylphenylsulfonyl)-S-arginin in 10 ml wasserfreiem Dichlormethan wird mit 603 mg (2,37 mmol) Bis-(2-oxo-3-oxazolidinyl)-phosphorylchlorid versetzt, worauf eine klare Lösung entsteht. Danach gibt man 1,33 ml (7,63 mmol) Ethyl-diisopropylamin zu, rührt 2 h bei -10 ° C nach und schüttet das Reaktionsgemisch in 60 ml 1 N $NaHCO_3$-Lösung. Die organische Phase wird abgetrennt, die Wasserphase wird mit 20 ml Dichlormethan extrahiert. Die vereinigten organischen Extrakte werden mit 50 ml Wasser gewaschen und über $MgSO_4$ getrocknet. Nach Abdampfen des Lösemittels im Vakuum und Chromatographie des Rückstandes an 42 g Kieselgel (Dichlormethan : Methanol 9:1) erhält man 692 mg (47%) der Titelverbindung als hellen Schaum.

$R_f$ = 0,25 (Dichlormethan: Methanol 9:1)

MS (FAB): m/z = 689 $(M+H)^+$

Wie für Beispiel 11 beschrieben erhält man durch Kupplung der Verbindungen aus Tabelle II mit den entsprechenden geschützten Argininderivaten in Gegenwart von Bis-(2-oxo-3-oxazolidinyl)-phosphorylchlorid die in Tabelle 2 aufgeführten Produkte:

Tabelle 2:

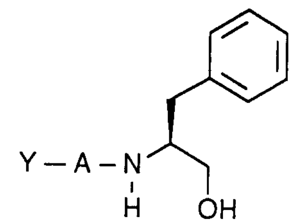

| Bsp.-Nr. | Y | A | Ausbeute (% d.Th.) | MS (FAB) m/z $(M+H)^+$ | $R_f$ / Laufmittel-verhältnis | Schmp. (°C) |
|---|---|---|---|---|---|---|
| 12 | Z-Arg(Tos)- | -N-$CH_3$-Ile | 51 | 723 | 0,13, I (9:1) | Schaum |
| 13 | Boc-Arg(Tos)- | -N-$CH_3$-Ala- | 46 | 647 | 0,30, I (9:1) | 102 |
| 14 | Boc-Arg(Tos)- | -N-$CH_3$-Gly- | 48 | 633 | 0,34, I (9:1) | 102 |
| 15 | Boc-Arg(Tos)- | -Aib- | 36 | 647 | 0,24, I (9:1) | 109 |
| 16 | Z-Arg(Tos)- | -Aib- | 48 | 681 | 0,19, I (9:1) | 86 |

In Analogie zu Beispiel 2 erhält man durch Abspaltung der tert.Butoxycarbonyl-Gruppe aus den Verbindungen der Tabellen 1 und 2 die in Tabelle 3 beschriebenen Dihydrochloride:

Tabelle 3:

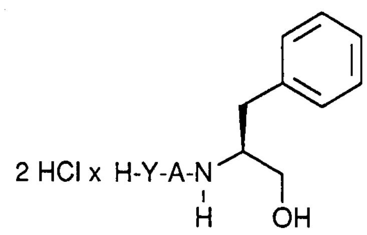

| Bsp.-Nr. | Y | -A- | Ausbeute (% d.Th.) | MS (FAB) m/z $(M+H)^+$ | $R_f$ / Laufmittel-verhältnis | Schmp. (Zers.) (°C) |
|---|---|---|---|---|---|---|
| 17 | -Arg(Tos) | -Gly(t-Bu)- | 97 | 575 | 0,34, I (8:2) | 189 |
| 18 | -Arg(Tos)- | -N-$CH_3$-Ile- | 90 | 589 | 0,01, I (9:1) | 118 |
| 19 | -Arg(Tos)- | -N-$CH_3$-Ala- | 93 | 547 | 0,12, I (85:15) | 122 |
| 20 | -Arg(Tos)- | -N-$CH_3$-Gly- | 94 | 533 | 0,08, I (85:15) | 118 |
| 21 | -Arg(Tos)- | -ß-Ala- | 98 | 533 | 0,06, I (85:15) | 130 |
| 22 | -Arg(Tos)- | -Aib- | 95 | 547 | 0,19, I (85:15) | 124 |

Beispiel 23

(2S)-2-{$N_\alpha$-[(3-iso-Propyloxycarbonyl-phenyl)aminocarbonyl]-$N^G$-(4-methylphenylsulfonyl)-S-arginyl-S-(tert.butyl)glycinyl}amino-3-phenyl-propan-1-ol

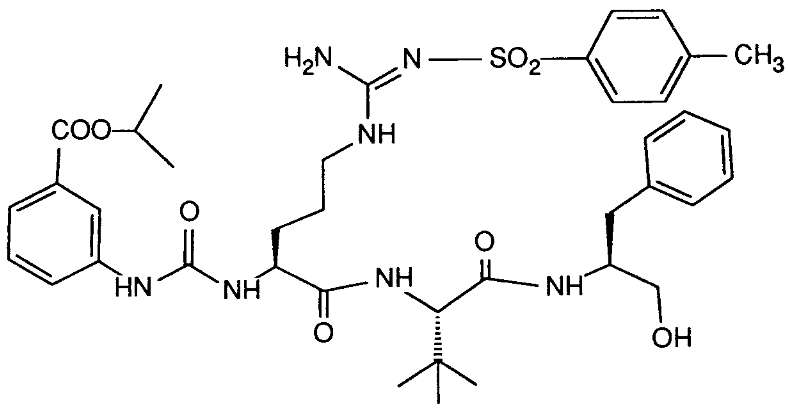

Eine gerührte Suspension von 1,30 g (2,00 mmol) (2S)-2-[$N^G$-(4-Methylphenylsulfonyl)-S-arginyl-S-(tert.butyl)glycinyl]amino-3-phenyl-propan-1-ol dihydrochlorid (Verbindung aus Beispiel 2) in 40 ml wasserfreiem Dichlormethan wird mit 0,64 ml (4,60 mmol) Triethylamin versetzt, wobei eine klare Lösung entsteht. Dazu gibt man 490 mg (2,40 mmol) 3-Isocyanato-benzoesäure-isopropylester und rührt 1 h bei Raumtemperatur nach. Man gibt 40 ml Toluol zu, engt die Reaktionsmischung im Vakuum ein und reinigt den Rückstand durch Chromatographie an 170 g Kieselgel (Dichlormethan : Methanol 95:5). Die produkthaltigen Fraktionen werden gesammelt und im Vakuum eingeengt. Der Rückstand wird mit 50 ml Ether verrieben, durch Filtration abgetrennt und im Hochvakuum getrocknet. Man erhält 1,01 g (64%) der Titelverbindung als farblose Kristalle.

Schmp.: 132 °C

$R_f$ = 0,08 (Dichlormethan: Methanol 9:1)

MS (FAB) m/z = 780 $(M+H)^+$, 802 $(M+Na)^+$.

Wie für Beispiel 23 beschrieben erhält man durch Reaktion der Verbindung aus Beispiel 2 mit den entsprechenden Isocyanaten die in der Tabelle 4 aufgeführten Verbindungen:

Tabelle 4:

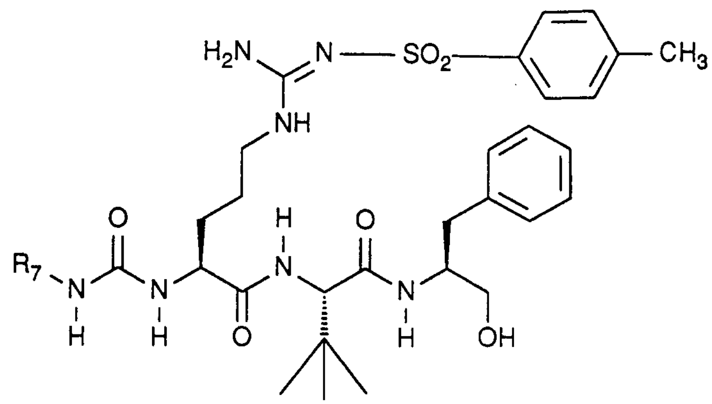

| Bsp.-Nr. | $R^7$ | Ausbeute (% d.Th.) | MS (FAB) m/z $(M+H)^+$ | $R_f$ / Laufmittel-verhältnis | Schmp. (Zers.) (°C) |
|---|---|---|---|---|---|
| 24 | 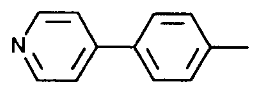 | 59 | 771 | 0,23, I (9:1) | 146 |
| 25 | 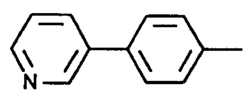 | 53 | 771 | 0,27, I (9:1) | 168 |
| 26 | 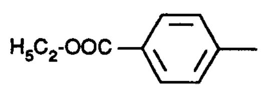 | 76 | 766 | 0,32, I (9:1) | 136 |
| 27 | 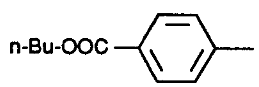 | 66 | 794 | 0,23, I (9:1) | 141 |

Fortsetzung Tabelle 4:

| Bsp.-Nr. | $R^7$ | Ausbeute (% d.Th.) | MS (FAB) m/z $(M+H)^+$ | $R_f$ / Laufmittel-verhältnis | Schmp. (Zers.) (°C) |
|---|---|---|---|---|---|
| 28 | 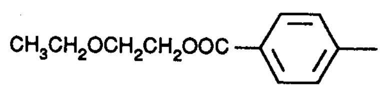 | 75 | 810 | 0,31, I (9:1) | 129 |
| 29 | 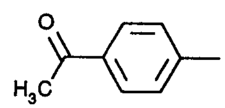 | 75 | 736 | 0,18, I (9:1) | 142 |

Beispiel 30

(2S)-2-[$N_\alpha$-Acetyl-$N^G$-(4-methyl-phenylsulfonyl)-S-arginyl-S-(tert.butyl)glycinyl)]amino-3-phenyl-propan-1-ol

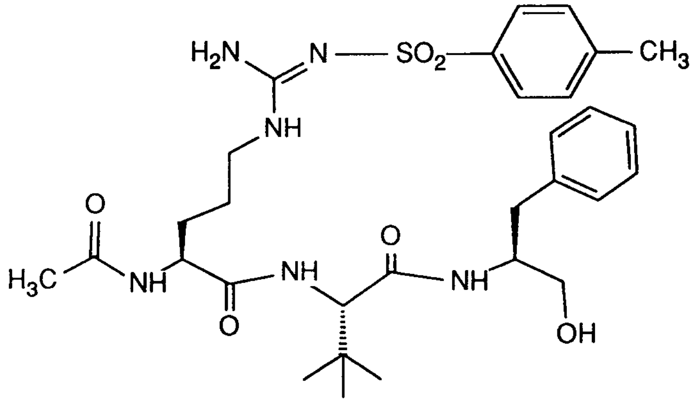

Zu einer auf 0°C gekühlten Lösung von 972 mg (1,5 mmol) der Verbindung aus Beispiel 2 in 6 ml wasserfreiem Dimethylformamid und 660 µl (6,00 mmol) N-Methylmorpholin werden 180 µl (1,86 mmol) Essigsäureanhydrid getropft. Nach 15 min bei 0°C wird in ein Gemisch aus 100 ml eiskalter Natriumbicarbonat-Lösung und 50 ml Ethylacetat gegossen und gut durchgerührt. Die organische Phase wird abgetrennt, die Wasserphase mit 10 ml Ethylacetat extrahiert und die verenigten organischen Extrakte über Magnesiumsulfat getrocknet. Nach Abdampfen des Lösemittels im Vakuum, Chromatographie des Rohproduktes an 45 g Kieselgel (Dichlormethan : Methanol 95:5) und Kristallisation aus 50 ml Dichlormethan : Ether 1:10 erhält man 394 mg (43%) der Titelverbindung als farblose Kristalle.
Schmp.: ab 114°C (Zers.)
$R_f$ = 0,24 (Dichlormethan: Methanol 9:1)
MS (FAB) m/z = 617 $(M+H)^+$
$^1$H-NMR (200 MHz, $CD_3OD$): $\delta$ = 0,93 (s, 9H, $(CH_3)_3$-C); 1,4-1,6 (m, 4H, $CH_2$); 1,95 (s, 3H, $CH_3CO$); 2,37 (s, 3H, $CH_3$); 2,68 (dd, J = 11, 14 Hz, 1H, $\underline{CH_2}Ph$); 2,90 (dd, J = 6, 14 Hz, 1H, $\underline{CH_2}Ph$); 3,12 (m, 2H, $CH_2N$); 3,49 (d, J = 6 Hz, 2H, $CH_2O$); 4,10 (m, $\overline{1H}$, NCH); 4,19 (s, NCHCO); 4,31 (m, $\overline{1H}$, NCH); 7,20 (m, 5H, Ph); 7,29, 7,73 (AB, J = 9,5 Hz, 4H, H arom.).

Beispiel 31

(2S)-2-[$N_\alpha$-Chinolin-2-carbonyl-$N^G$-(4-methyl-phenylsulfonyl)-S-arginyl-S-(tert.-butyl)glycinyl]amino-3-phenyl-propan-1-ol

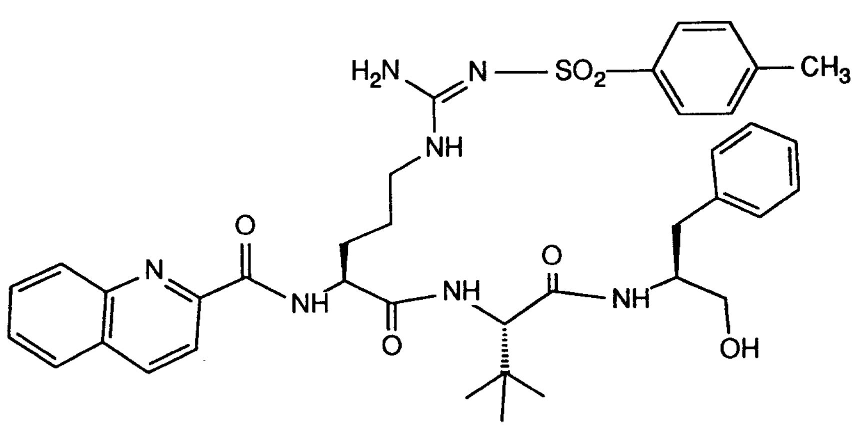

Eine auf 0°C gekühlte, gerührte Lösung von 381 mg (2,20 mmol) Chinolin-2-carbonsäure und 337 mg (2,20 mmol) HOBT in 30 ml wasserfreiem Dichlormethan wird mit 433 mg (2,10 mmol) DCC versetzt und 5 min

gerührt. Danach wird eine Lösung von 1,29 g (2,00 mmol) der Verbindung aus Beispiel 2 und 0,77 ml (7,00 mmol) N-Methylmorpholin in 30 ml Dichlormethan zugetropft. Das Kühlbad wird entfernt und die Reaktionsmischung darf 2 h bei Raumtemperatur rühren. Das Ende der Reaktion wird dünnschichtchromatographisch festgestellt. Man trennt den entstandenen Harnstoff durch Filtration ab, engt das Filtrat im Vakuum ein und reinigt das Rohprodukt durch Chromatographie an 80 g Kieselgel (Dichlormethan : Methanol 95:5). Man erhält 1,14 g (78% der Theorie) der Titelverbindung als farblose Kristalle.
Schmp.: 131 °C
$R_f$ = 0,27 (Dichlormethan : Methanol 9:1)
MS (FAB) m/z = 730 $(M+H)^+$
Wie für Beispiel 31 beschrieben erhält man durch Kondensation der Verbindung aus Beispiel 2 die in Tabelle 5 aufgeführten Produkte:

Tabelle 5:

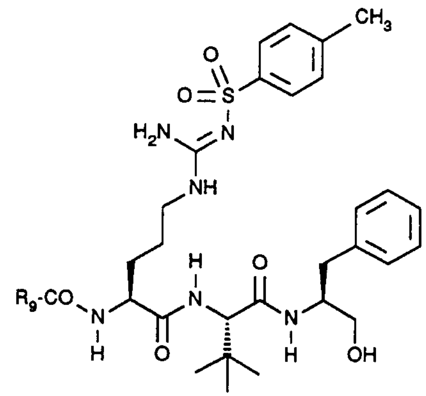

| Bsp.-Nr. | $R^9$ | Ausbeute (% d.Th.) | MS (FAB) m/z $(M+H)^+$ | $R_f$ / Laufmittel-verhältnis | Schmp. (Zers.) (°C) |
|---|---|---|---|---|---|
| 32 | 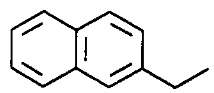 | 39 | 743 | 0,27, I (9:1) | 130 |
| 33 | 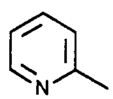 | 62 | 680 | 0,32, I (9:1) | 114 |
| 34 | 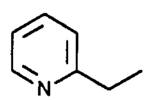 | 44 | 694 | 0,29, I (9:1) | 121 |
| 35 | 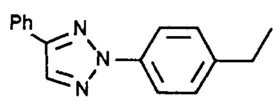 | 71 | 836 | 0,30, I (9:1) | 125 |

<u>Fortsetzung Tabelle 5:</u>

| Bsp.-Nr. | $R^9$ | Ausbeute | MS (FAB) | $R_f$ / Laufmittel- | Schmp. (Zers.) |
|---|---|---|---|---|---|
| 36 | 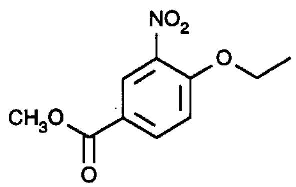 | 72 | 812 | 0,33, I (9:1) | 130 |
| 37 | 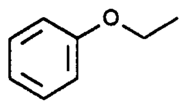 | 68 | 709 | 0,29, I (9:1) | 105 |
| 38 | 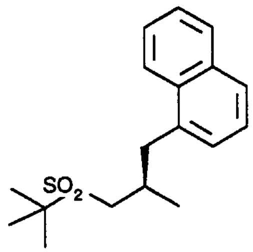 | 65 | 891 | 0,36, I (9:1) | 147 |
| 39 | 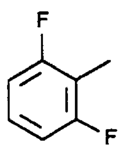 | 65 | 715 | 0,32, I (9:1) | 128 |
| 40 | 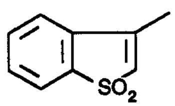 | 17 | 767 | 0,28, I (9:1) | 139 |

Beispiel 41

(2S)-2-($N_\alpha$-(2,6-Dichlor-phenyl-methoxycarbonyl)-$N^G$-(4-methyl-phenylsulfonyl)-S-arginyl-S-(tert.butyl)-glycinyl]amino-3-phenyl-propan-1-ol

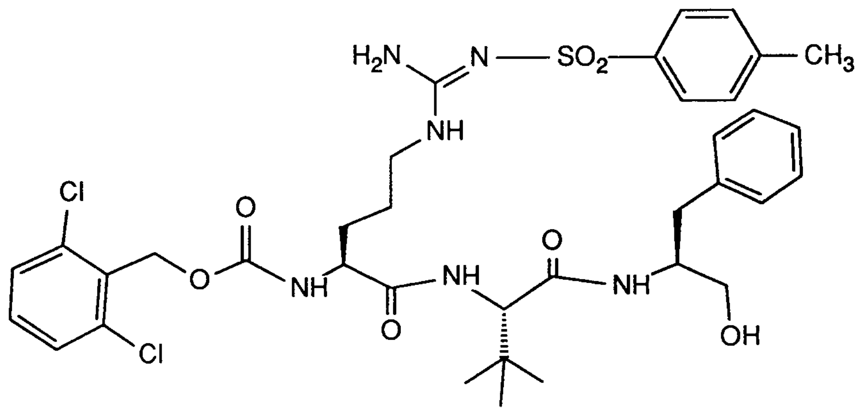

Eine auf 0 °C gekühlte, gerührte Lösung von 1,30 g (2,00 mmol) der Verbindung aus Beispiel 2 in 9 ml Dioxan und 6 ml Wasser wird innerhalb von 2 h portionsweise mit 551 mg (2,30 mmol) 2,6-Dichlor-benzyloxycarbonylchlorid versetzt, wobei durch gleichzeitige Zugabe einer 2 N wässriger NaOH-Lösung pH 9-10 gehalten wird. Danach wird in ein Gemisch von 15 ml Eiswasser, 6 ml 1 N Zitronensäure und 30 ml Ethylacetat eingerührt. Die organische Phase wird abgetrennt und die Wasserphase wird mit 5 mal 20 ml Ethylacetat extrahiert. Die vereinigten organischen Extrakte werden über $MgSO_4$ getrocknet. Nach Abdampfen des Lösemittels im Vakuum, Chromatographie des Rückstands an 75 g Kieselgel (Dichlormethan: Methanol 95:5) und Kristallisation des Produkts aus 50 ml Ether erhält man 960 mg (61%) der Titelverbindung als farblose Kristalle.
Schmp.: ab 121 °C (Zers.)
$R_f$ = 0,43 (Dichlormethan: Methanol 9:1)
MS (FAB) m/z = 777 $(M+H)^+$
$^1$H-NMR (200 MHz, $CD_3OD$) $\delta$ = 0,96 (s, 9H, $[CH_3]_3C$); 1,4 - 1,6 (m, 4H, $CH_2$); 2,37 (s, 3H, $CH_3$); 2,69 (dd, J = 9, 13 Hz, 2H, $\underline{CH_2}Ph$); 2,90 (dd, J = 6, 13 Hz, 2H, $\underline{CH_2}Ph$); 3,12 (m, 2H, $CH_2N$); 3,50 (d, J = 5 Hz, 2H, $CH_2O$); 4,10 (m, 2H, NCH); 4,20 (s, 1H, NCHCO); 5,36 (s, 2H, $CH_2OCO$); 7,15 - 7,45 (m, 8H, H arom.); 7,28, 7,72 (AB, J = 8,5 Hz, 4H, H arom.)

Wie für Beispiel 41 beschrieben erhält man durch Umsetzung der Verbindung aus Beispiel 2 mit den entsprechenden Benzyloxycarbonylchloriden die in Tabelle 6 aufgeführten Verbindungen:

Tabelle 6:

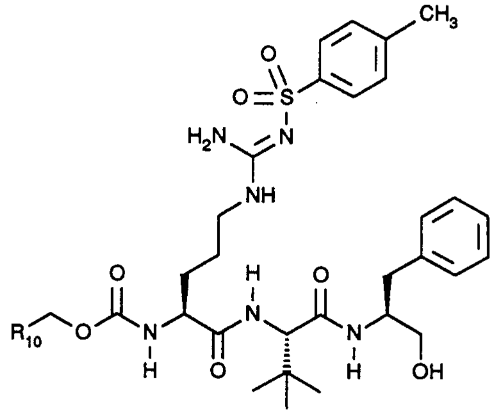

| Bsp.-Nr. | $R^{10}$ | Ausbeute (% d.Th.) | MS (FAB) m/z $(M+H)^+$ | $R_f$ / Laufmittel-verhältnis | Schmp. (Zers.) (°C) |
|---|---|---|---|---|---|
| 42 | $C_6H_5$- | 64 | 709 | 0,29, I (9:1) | 110 |
| 43 | 4-$NO_2$-$C_6H_4$- | 68 | 754 | 0,39, I (9:1) | 115 |
| 44 | 2,5-$CH_3$-$C_6H_3$- | 42 | 737 | 0,21, I (9:1) | 105 |

Beispiel 45

(2S)-2-[$N_\alpha$-(3-Pyridinyl-methoxycarbonyl)-$N^G$-(4-methyl-phenylsulfonyl)-S-arginyl-S-(tert.butyl)glycinyl]amino-3-phenyl-propan-1-ol

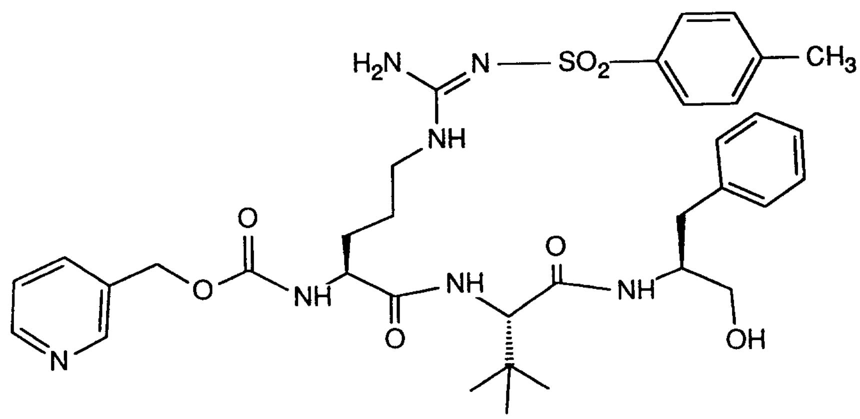

Eine gerührte Suspension von 1,30 g (2,00 mmol) der Verbindung aus Beispiel 2 und 1.10 g (4,00 mmol) 4-Nitrophenyl-3-pyridinylmethylcarbonat [hergestellt nach D.F. Veber et al., J. Org. Chem. 42, 3286 (1977)] in 10 ml Dioxan und 10 ml Wasser wird durch stetige Zugabe von wässriger 2 N NaOH-Lösung (Bedarf ca. 2,5 ml) auf pH 7,5 gehalten und 15 h bei Raumtemperatur gerührt. Das Ende der Reaktion wird dünnschichtchromatographisch festgestellt, dann wird die Reaktionsmischung in ein Gemisch aus 25 ml 1 N Zitronensäure und 20 ml Ethylacetat eingerührt. Die wässrige Phase wird abgetrennt, durch Zugabe von 2 N NaOH auf pH 9 gestellt und mit 20 ml Ethylacetat (3 mal) extrahiert. Die vereinigten organischen Extrakte werden über $MgSO_4$ getrocknet. Das Lösemittel wird im Vakuum abgedampft und der Rückstand aus Dichlormethan/Ether kristallisiert. Man erhält 1,15 g (81%) der Titelverbindung als farblose Kristalle.
Schmp.: ab 88 °C (Zers.)
$R_f$ = 0,12 (Dichlormethan: Methanol 9:1)
MS (FAB) m/z = 710 $(M+H)^+$
$^1$H-NMR (200 MHz, $CD_3OD$) $\delta$ = 0,92 (s, 9H, $[CH_3]_3C$); 1,4 - 1,6 (m, 4H, $CH_2$); 2,37 (s, 3H, $CH_3$); 2,68 (dd, J = 8, 14 Hz, 1H, $\underline{CH_2}Ph$); 2,90 (dd, J = 6, 14 Hz, 1H, $\underline{CH_2}Ph$); 3,12 (m, 2H, $CH_2N$); 3,50 (m, 2H, $CH_2O$); 4,10 (m, 2H, CH); 4,18 (s, 1H, NCHCO); 5,15 (s, 2H, $CH_2\overline{OCO}$); 7,18 (m, 5H, Ph); 7,27, 7,72 (AB, J = 8 Hz, 4H, H arom.); 7,41 (dd, J = 5, 7 Hz, 1H, 5-Pyridyl-H); 7,85 (m, 1H, 4-Pyridyl-H); 8,45 (dd, J = 1,5 Hz, 6-Pyridyl-H); 8,55 (d, J = 1 Hz, 1H, 2-Pyridyl-H).

Wie für Beispiel 45 beschrieben erhält man durch Umsetzung der Verbindung aus Beispiel 2 mit den entsprechenden 4-Nitrophenylcarbonaten die in Tabelle 7 aufgeführten Verbindungen:

Tabelle 7:

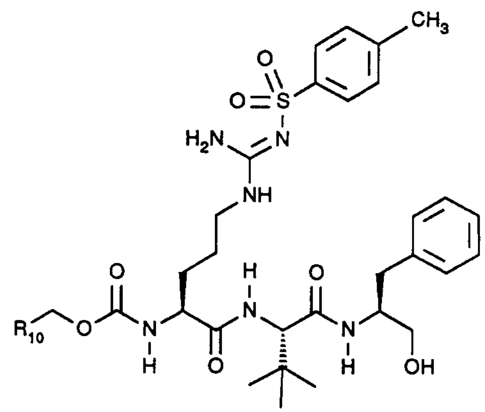

| Bsp.-Nr. | $R^{10}$ | Ausbeute (% d.Th.) | MS (FAB) m/z $(M+H)^+$ | $R_f$ / Laufmittelverhältnis | Schmp. (Zers.) (°C) |
|---|---|---|---|---|---|
| 46 | 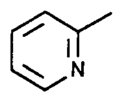 | 2 | 710 | 0,27, I (9:1) | 98 |
| 47 | 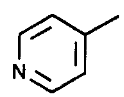 | 25 | 710 | 0,28, I (9:1) | 120 |
| 48 | 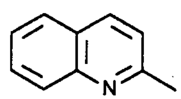 | 11 | 760 | 0,35, I (9:1) | amorph |
| 49 | 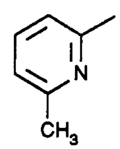 | 17 | 724 | 0,30, I (9:1) | Schaum |

Beispiel 50

(2R,S)-2-[$N_\alpha$-(Benzyloxycarbonyl)-$N^G$-(4-methyl-phenylsulfonyl)-S-arginyl-S-(tert.butyl)glycinyl]amino-3-phenyl-propan-1-al

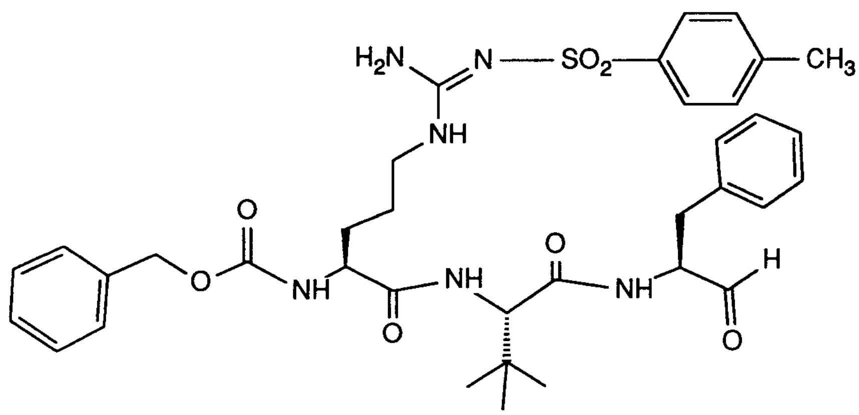

Eine Lösung von 218 mg (0,31 mmol) der Verbindung aus Beispiel 8 in 2,5 ml wasserfreiem DMSO und 0,39 ml (2,77 mmol) Triethylamin wird mit 221 mg (1,39 mmol) Pyridin-Schwefeltrioxid-Komplex versetzt und 1 h bei Raumtemperatur gerührt. Danach wird das Reaktionsgemisch in 20 ml Ether eingerührt. Das Gemisch darf kurze Zeit stehen, wobei sich ein Öl abscheidet. Die Etherphase wird abdekantiert, und das Öl wird in 5 ml Toluol aufgenommen. Das Toluol wird im Vakuum eingedampft, und der Rückstand wird an 43 g Kieselgel (Dichlormethan: Methanol 95.5) chromatographiert. Man erhält 195 mg (89%) der Titelverbindung als hellgelbes Öl (Diastereomerengemisch).
$R_f$ = 0,25, 0,33 (Dichlormethan: Methanol 9:1)
MS (FAB): m/z = 707 $(M+H)^+$
Wie für Beispiel 50 beschrieben erhält man durch Oxidation der Alkohole aus vorgenannten Tabellen die in den Tabelle 8 und 9 aufgeführten Verbindungen als Gemisch der diastereomeren Aldehyde:

Tabelle 8:

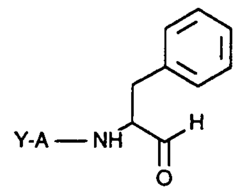

| Bsp.-Nr. | Y- | A | Ausbeute (% d.Th.) | MS (FAB) m/z $(M+H)^+$ | $R_f$ / Laufmittel-verhältnis | Schmp. (Zers.) (°C) |
|---|---|---|---|---|---|---|
| 51 | Boc-Arg(Tos)- | - | 57 | 560 | 0,69, I (85:15) | 99 |
| 52 | Boc-Arg(Tos)- | Gly(t-Bu)- | 76 | 673 | 0,22, 0,27, II (9:1) | Öl |
| 53 | Z-Arg(Tos)- | Gly(t-Bu)- | 96 | 707 | 0,39, 0,44, I (9:1) | Schaum |
| 54 | Boc-Arg(Tos)- | N-$CH_3$-Ile- | 56 | 687 | 0,14, I (9:1) | Schaum |
| 55 | Z-Arg(Tos)- | N-$CH_3$-Ile- | 47 | 721 | 0,20, I (9:1) | Schaum |
| 56 | Boc-Arg(Tos)- | Ile | 55 | 673 | 0,41, 0,44, I (9:1) | Schaum |
| 57 | Boc-Arg(Tos)- | N-$CH_3$-Ala- | 72 | 645 | 0,41, 0,45, I (9:1) | Schaum |
| 58 | Boc-Arg(Tos)- | N-$CH_3$-Gly- | 89 | 631 | 0,40, I (9:1) | Schaum |
| 59 | Boc-Arg(Tos)- | ß-Ala- | 66 | 631 | 0,30, I (9:1) | Schaum |
| 60 | Boc-Arg(Tos)- | Aib- | 90 | 645 | 0,13, I (9:1) | Öl |
| 61 | Z-Arg(Tos)- | Aib- | 60 | 679 | 0,53, I (9:1) | Schaum |

Tabelle 9:

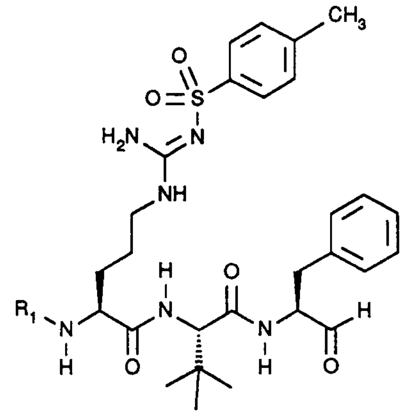

| Bsp.-Nr. | $R^1$ | Ausbeute (% d.Th.) | MS (FAB) m/z $(M+H)^+$ | $R_f$ / Laufmittelverhältnis | Schmp. (Zers.) (°C) |
|---|---|---|---|---|---|
| 62 | 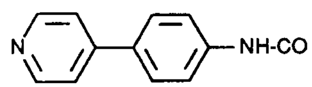 | 22 | 769 | 0,17, 0,21, I (9:1) | 151 (Zers.) |
| 63 | 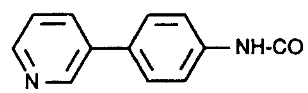 | 37 | 769 | 0,22, 0,31, (9:1) | 100 (Zers.) |
| 64 | 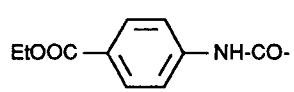 | 54 | 764 | 0,29, 0,33 (9:1) | 121 |
| 65 | 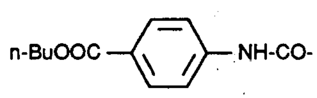 | 55 | 792 | 0,29, 0,33 (9:1) | 120 |

Fortsetzung Tabelle 9:

| Bsp.-Nr. | $R^1$ | Ausbeute (% d.Th.) | MS (FAB) m/z $(M+H)^+$ | $R_f$ / Laufmittelverhältnis | Schmp. (Zers.) (°C) |
|---|---|---|---|---|---|
| 66 | 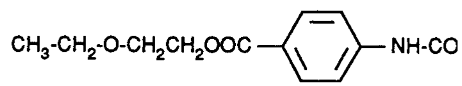 | 63 | 808 | 0,24, 0,28, I (9:1) | 112 |
| 67 | 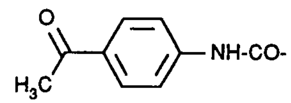 | 62 | 734 | 0,37, 0,42, I (9:1) | 137 |
| 68 | 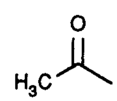 | 54 | 615 | 0,23, I (9:1) | 122 (Zers.) |
| 69 | 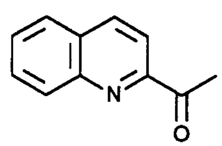 | 88 | 728 | 0,43, 0,49, I (9:1) | Schaum 131 (Zers.) 3) |
| 70 | 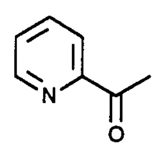 | 87 | 678 | 0,36, 0,39, I (19:1) | Schaum |

Fortsetzung Tabelle 9:

| Bsp.-Nr. | $R^1$ | Ausbeute (% d.Th.) | MS (FAB) m/z $(M+H)^+$ | $R_f$ / Laufmittelverhältnis | Schmp. (Zers.) (°C) |
|---|---|---|---|---|---|
| 71 | 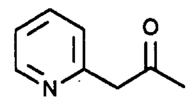 | 59 | 692 | 0,30, 0,40, I (9:1) | 115 (Zers.) |
| 72 | 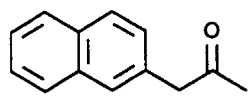 | 79 | 741 | 0,39, 0,41, I (9:1) | Öl |
| 73 | 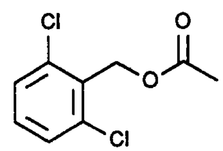 | 96 | 775 | 0,26, 0,31, I (9:1) | Schaum |
| 74 | 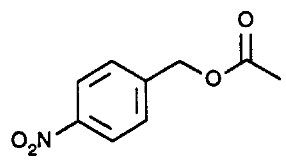 | 87 | 752 | 0,41, 0,46, I (9:1) | Schaum |
| 75 | 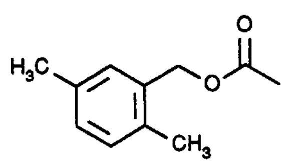 | 36 | 735 | 0,35, 0,39, I (19:1) | Schaum |

Fortsetzung Tabelle 9:

| Bsp.-Nr. | $R^1$ | Ausbeute (% d.Th.) | MS (FAB) m/z $(M+H)^+$ | $R_f$ / Laufmittel-verhältnis | Schmp. (Zers.) (°C) |
|---|---|---|---|---|---|
| 76 | 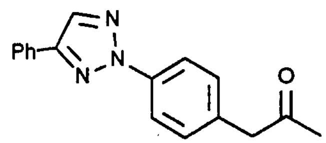 | 81 | 940 [2)] | 0,36, 0,39, I (9:1) | Schaum |
| 77 | 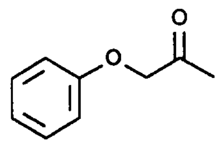 | 96 | 707 | 0,39, 0,44, I (9:1) | Schaum |
| 78 | 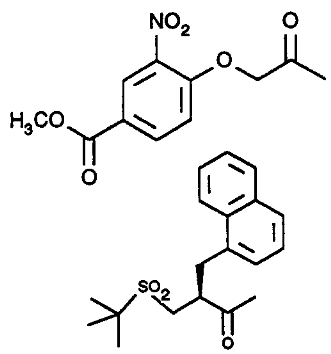 | 37 | 810 | 0,45, 0,50, I (9:1) | 105 (Zers.) |
| 79 | | 48 | 889 | 0,46, I (9:1) | 128 (Zers.) |
| 80 | 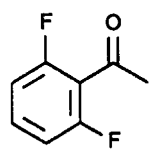 | 96 | 713 | 0,53, 0,57, I (19:1) | Schaum |

Fortsetzung Tabelle 9:

| Bsp.-Nr. | $R^1$ | Ausbeute (% d.Th.) | MS (FAB) m/z $(M+H)^+$ | $R_f$ / Laufmittel-verhältnis | Schmp. (Zers.) (°C) |
|---|---|---|---|---|---|
| 81 | 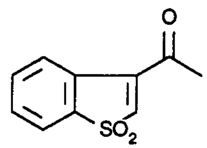 | 49 | 765 | 0,39, 0,42, I (9:1) | Öl |
| 82 | 2 HCl x 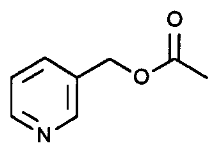 | 44 | 708 | 0,23, 0,26, I (9:1) | 112 (Zers.) 3) |
| 83 | 2 HCl x 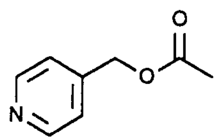 | 30 | 708 | 0,24, 0,28, I (9:1) | 98 (Zers.) 3) |
| 84 | 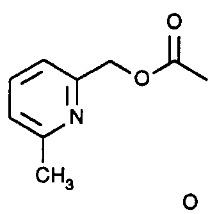 | 37 | 722 | 0,26, 0,30, I (9:1) | Schaum |
| 85 | 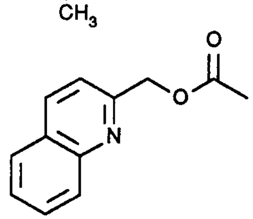 | 51 | 758 | 0,33, 0,36, I (9:1) | amorph |

3) = Hydrochlorid

Beispiel 86

(2R,S)-2-{$N_\alpha$-[(4-(3-Pyridinyl)-phenyl)aminocarbonyl]-$N^G$-(4-methylphenylsulfonyl)-S-arginyl-S-(tert.butyl)-glycinyl}-amino-3-phenyl-propan-1al Dihydrochlorid

2 HCl x

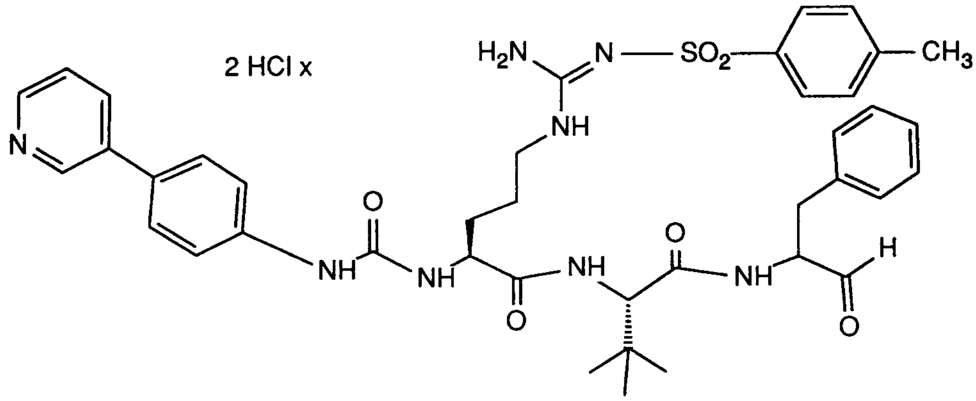

Zu einer gerührten Lösung von 226 mg (0,29 mmol) der Verbindung aus Beispiel 63 in 4 ml wasserfreiem Dioxan, tropft man 4 ml einer 1 N Lösung von gasförmigem Chlorwasserstoff in Ether. Man rührt 30 min bei Raumtemperatur, gibt 20 ml Ether zu, trennt den Niederschlag durch Filtration ab und trocknet im Hochvakuum. Man erhält 205 mg (81%) der Titelverbindung als farblose Kristalle (Gemisch der diastereomeren Aldehyde).
Schmp.: ab 98 °C (Zers.)
$R_f$ = 0,22, 0,33 (Dichlormethan: Methanol 9:1)
MS (FAB) m/z = 769 $(M+H)^+$
In Analogie zur Vorschrift des Beispiels 45 und den Erläuterungen zu denen in Tabelle 7 aufgeführten Verbindungen werden die Beispiele aus Tabelle 10 hergestellt:

Tabelle 10:

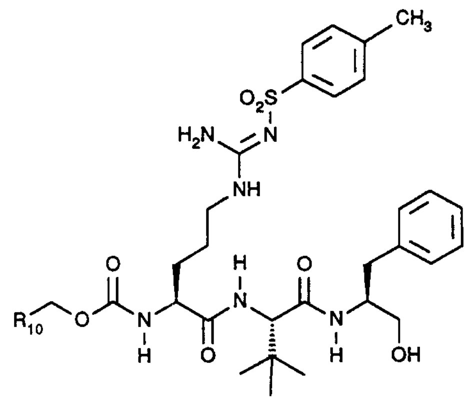

| Bsp.-Nr. | $R^{10}$ | Ausbeute (% d.Th.) | MS (FAB) m/z $(M+H)^+$ | $R_f$ / Laufmittelverhältnis | F°C(Zers.) |
|---|---|---|---|---|---|
| 87 | 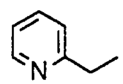 | 22 | 724 | 0,23, I (9:1) | 106 |
| 88 | 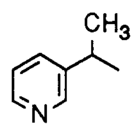 | 30 | 724 | 0,27, I (9:1) | 124(Zers.) |
| 89 | 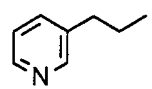 | 45 | 738 | 0,32, I (9:1) | 100(Zers.) |
| 90 | 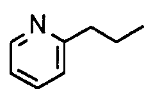 | 31 | 738 | 0,30, I (9:1) | 116(Zers.) |
| 91 | 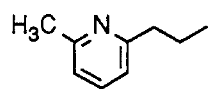 | 46 | 752 | 0,29, I (9:1) | 86(Zers.) |

In Analogie zur Vorschrift des Beispiels 50 und den Erläuterungen zu den Verbindungen aus den Tabellen 8 und 9 werden die Beispiele aus Tabelle 11 aufgeführten Beispiele hergestellt:

Tabelle 11:

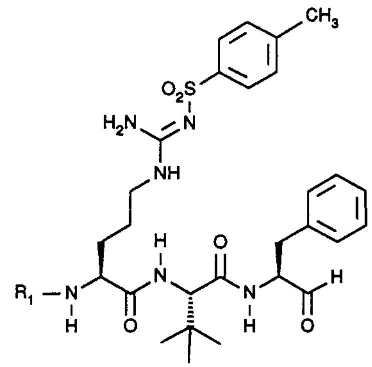

| Bsp.-Nr. | R[1] | Ausbeute (% d.Th.) | MS (FAB) m/z $(M+H)^+$ | $R_f$ / Laufmittel-verhältnis | F°C (Zers.) |
|---|---|---|---|---|---|
| 92 2 x HCl | 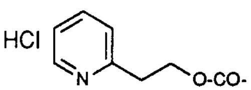 | 66 | 722 | 0,25/0,27, I (9:1) | 145 (Zers.)[3)] |
| 93 | 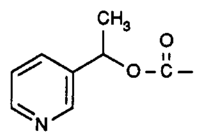 | 65 | 722 | 0,26/0,29, I (9:1) | 108(Zers.) |
| 94 | 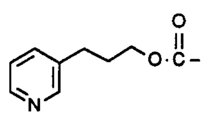 | 33 | 736 | 0,30/0,33, I (9:1) | amorph |
| 95 2 x HCl | 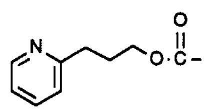 | 17 | 736 | 0,22/0,25, I (9:1) | Lyophilisat |
| 96 2 x HCl | 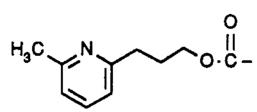 | 28 | 750 | 0,41/0,43, I (9:1) | Lyophilisat |

3) ≤ Hydrochlorid

**Patentansprüche**

**1.** Pseudopeptide der allgemeinen Formel (I)

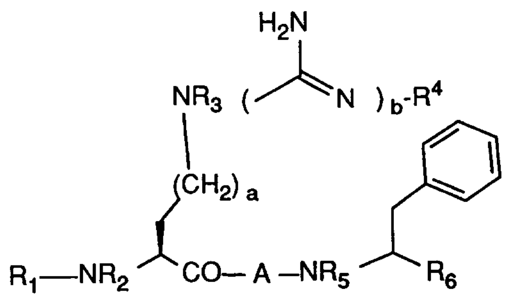

(I)

in welcher

a für eine Zahl 1, 2 oder 3 steht,

b für eine Zahl 0 oder 1 steht,

$R^1$ für Wasserstoff oder für eine Aminoschutzgruppe steht, oder
für einen Rest der Formel $R^7$-$NR^8$-CO-, $R^9$-$(CH_2)_c$-CO-, $R^{10}$-$(CH_2)_d$-O-CO oder für einen Rest der Formel -$SO_2$-$R^{11}$ steht,
worin

$R^7$ Cycloalkyl mit 3 bis 6 Kohlenstoffatomen bedeutet oder geradkettiges oder verzweigtes Alkyl mit bis zu 18 Kohlenstoffatomen bedeutet, das gegebenenfalls durch Hydroxy, geradkettiges oder verzweigtes Alkoxy mit bis zu 4 Kohlenstoffatomen, Halogen, Trifluormethyl, Trifluormethoxy, Cycloalkyl mit 3 bis 6 Kohlenstoffatomen oder durch Aryl mit 6 bis 10 Kohlenstoffatomen substituiert ist, das seinerseits bis zu 2-fach gleich oder verschieden durch Carboxy, Cyano, Hydroxy, Halogen, Perhalogenalkyl mit bis zu 5 Kohlenstoffatomen oder durch geradkettiges oder verzweigtes Acyl, Alkoxy oder Alkoxycarbonyl mit jeweils bis zu 6 Kohlenstoffatomen substituiert sein kann, oder
Alkyl gegebenenfalls durch eine Gruppe der Formel -$CO_2R^{12}$ substituiert ist,
worin

$R^{12}$ Wasserstoff oder geradkettiges oder verzweigtes Alkyl oder Alkenyl mit jeweils bis zu 8 Kohlenstoffatomen bedeutet, die gegebenenfalls durch Phenyl substituiert sind,
oder

$R^7$ Aryl mit 6 bis 10 Kohlenstoffatomen bedeutet, das gegebenenfalls bis zu 3-fach gleich oder verschieden durch Carboxy, Amino, Halogen, Hydroxy, Cyano, Perhalogenalkyl mit bis zu 5 Kohlenstoffatomen oder durch geradkettiges oder verzweigtes Acyl, Alkoxy, Vinylalkoxycarbonyl, Alkoxycarbonyl oder mit jeweils bis zu 6 Kohlenstoffatomen substituiert ist, das seinerseits durch geradkettiges oder verzweigtes Alkoxy mit bis zu 6 Kohlenstoffatomen substituiert ist, oder
einen Aminosäurerest der Formel

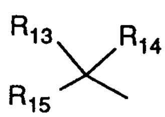

bedeutet,
worin

$R^{13}$ und $R^{14}$ gleich oder verschieden sind und Wasserstoff oder Methyl bedeuten, oder

$R^{13}$ und $R^{14}$ gemeinsam einen 5- oder 6-gliedrigen gesättigten carbocyclischen Ring bilden,
oder

$R^{13}$ Wasserstoff oder Methyl bedeutet
und

$R^{14}$ Cycloalkyl mit 3 bis 8 Kohlenstoffatomen oder Aryl mit 6 bis 10 Kohlenstoffatomen oder Wasserstoff bedeutet, oder
geradkettiges oder verzweigtes Alkyl mit bis zu 8 Kohlenstoffatomen bedeutet,

wobei das Alkyl gegebenenfalls durch Methylthio, Hydroxy, Mercapto, Guanidyl oder durch eine Gruppe der Formel $-NR^{16}R^{17}$ oder $R^{18}$-OC- substituiert ist,

worin

$R^{16}$ und $R^{17}$ unabhängig voneinander Wasserstoff, geradkettiges oder verzweigtes Alkyl mit bis zu 8 Kohlenstoffatomen oder Phenyl bedeuten,

und

$R^{18}$ Hydroxy, Benzyloxy, Alkoxy mit bis zu 6 Kohlenstoffatomen oder die oben aufgeführte Gruppe $-NR^{16}R^{17}$ bedeutet,

$R^{15}$ geradkettiges oder verzweigtes Alkyl mit bis zu 8 Kohlenstoffatomen bedeutet, das gegebenenfalls durch Hydroxy oder geradkettiges oder verzweigtes Alkoxy mit bis zu 6 Kohlenstoffatomen substituiert ist, oder Carboxy, Allyloxycarbonyl, geradkettiges oder verzweigtes Alkoxycarbonyl mit bis zu 8 Kohlenstoffatomen oder Benzyloxycarbonyl bedeutet,

oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^{16}R^{17}$ substituiert ist,

worin

$R^{16}$ und $R^{17}$ die oben angegebene Bedeutung haben,

oder das Alkyl gegebenenfalls durch einen 5- bis 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,

oder

$R^7$ einen Rest der Formel

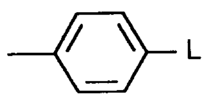

bedeutet,

worin

L Phenyl oder Pyridyl bedeutet

$R^8$ Wasserstoff, geradkettiges oder verzweigtes Alkyl mit bis zu 6 Kohlenstoffatomen oder eine Aminoschutzgruppe bedeutet,

$R^9$ geradkettiges oder verzweigtes Alkyl mit bis zu 8 Kohlenstoffatomen, welches durch Pyridyl substituiert sein kann, bedeutet, oder Aryloxy oder Aryl mit jeweils 6 bis 10 Kohlenstoffatomen, Indolyl, Chinolyl, Chinoxalinyl, Isochinolyl oder einen 5- bis 7-gliedrigen, gesättigten oder ungesättigten Heterocyclus mit bis zu 3 Heteroatomen aus der Reihe S, N oder O bedeutet, wobei die Cyclen bis zu 3-fach gleich oder verschieden durch Carboxy, Cyano, Hydroxy, Halogen, Amino, Nitro, Methylamino, Perhalogenalkyl mit bis zu 5 Kohlenstoffatomen oder durch geradkettiges oder verzweigtes Alkyl, Acyl, Alkoxy oder Alkoxycarbonyl mit jeweils bis zu 6 Kohlenstoffatomen substituiert sein können,

oder Aryl gegebenenfalls auch durch einen 5- bis 7-gliedrigen, gesättigten oder ungesättigten Heterocyclus mit bis zu 3 Heteroatomen aus der Reihe S, N oder O substituiert ist, das seinerseits durch Phenyl substituiert sein kann,

oder

$R^9$ einen Rest der Formel

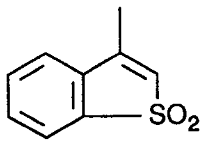 , 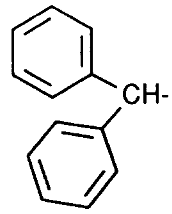 ,

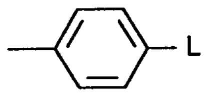 oder 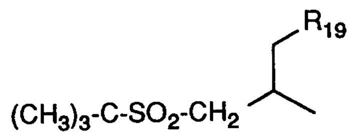

bedeutet,

worin

L' — die oben angegebene Bedeutung von L hat und mit dieser gleich oder verschieden ist,

$R^{19}$ — Phenyl oder Naphthyl bedeutet,

c — eine Zahl 0, 1, 2 oder 3 bedeutet,

d — eine Zahl 0, 1, 2 oder 3 bedeutet,

$R^{10}$ — die oben angegebene Bedeutung von $R^9$ hat und mit dieser gleich oder verschieden ist,

$R^{11}$ — Methyl, Phenyl oder Naphthyl bedeutet, das gegebenenfalls bis zu 4-fach gleich oder verschieden durch Methyl oder Methoxy substituiert ist, oder einen Rest der Formel

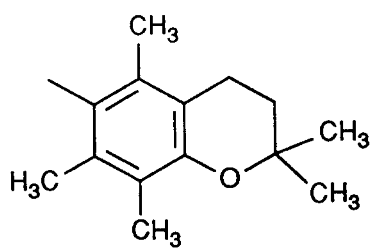

bedeutet,

$R^2$, $R^3$ und $R^5$ — gleich oder verschieden sind und für Wasserstoff, geradkettiges oder verzweigtes Alkyl mit bis zu 4 Kohlenstoffatomen oder für eine Aminoschutzgruppe stehen,

$R^4$ — für Wasserstoff, für Nitro, für eine Aminoschutzgruppe oder für einen Rest der Formel $-SO_2-R^{20}$ steht,

worin

$R^{20}$ — die oben angegebene Bedeutung von $R^{11}$ hat und mit dieser gleich oder verschieden ist,

A — für eine Bindung oder für einen Rest der Formel

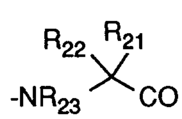

steht,

worin

$R^{21}$ und $R^{22}$ — gleich oder verschieden sind und die oben angegebene Bedeutung von $R^{13}$ und $R^{14}$ haben und mit dieser gleich oder verschieden sind,

$R^{23}$ die oben angegebene Bedeutung von $R^2$, $R^3$ oder $R^5$ hat und mit dieser gleich oder verschieden ist,

$R^6$ für Formyl oder Carboxy steht oder
für geradkettiges oder verzweigtes Alkoxycarbonyl mit bis zu 8 Kohlenstoffatomen steht, oder
für einen Rest der Formel $-CH_2-OR^{24}$ oder $-CH(OR^{25})_2$ steht,
worin

$R^{24}$ und $R^{25}$ gleich oder verschieden sind und Wasserstoff, geradkettiges oder verzweigtes Alkyl mit bis zu 6 Kohlenstoffatomen oder eine Hydroxyschutzgruppe bedeuten,

und deren Salze,
mit der Maßgabe, daß A nicht für Valin stehen darf.

**2.** Verbindungen der allgemeinen Formel (I), gemäß Anspruch 1
in welcher

a für eine Zahl 2 oder 3 steht,

b für eine Zahl 0 oder 1 steht,

$R^1$ für Wasserstoff, tert.Butoxycarbonyl (Boc), Benzyloxycarbonyl (Z) oder 9-Fluorenylmethoxycarbonyl (FMOC) steht, oder
für einen Rest der Formel $R^7-NR^8-CO-$, $R^9-(CH_2)_c-CO-$, $R^{10}-(CH_2)_d-O-CO$ oder für einen Rest der Formel $-SO_2-R^{11}$ steht,
worin

$R^7$ Cyclopentyl oder Cyclohexyl bedeutet oder geradkettiges oder verzweigtes Alkyl mit bis zu 16 Kohlenstoffatomen bedeutet, das gegebenenfalls durch Hydroxy, Methoxy, Fluor, Chlor, Brom, Trifluormethyl, Trifluormethoxy, Cyclopentyl, Cyclohexyl oder Phenyl substituiert ist, das seinerseits bis zu 2-fach gleich oder verschieden durch Carboxy, Cyano, Hydroxy, Fluor, Chlor, Brom, Perhalogenalkyl mit bis zu 4 Kohlenstoffatomen oder durch geradkettiges oder verzweigtes Acyl, Alkoxy oder Alkoxycarbonyl mit jeweils bis zu 4 Kohlenstoffatomen substituiert sein kann, oder
Alkyl gegebenenfalls durch eine Gruppe der Formel $-CO_2R^{12}$ substituiert ist,
worin

$R^{12}$ Wasserstoff oder geradkettiges oder verzweigtes Alkyl oder Alkenyl mit jeweils bis zu 6 Kohlenstoffatomen bedeutet, die gegebenenfalls durch Phenyl substituiert sind,
oder

$R^7$ Phenyl oder Naphthyl bedeutet, das gegebenenfalls bis zu 3-fach gleich oder verschieden durch Carboxy, Amino, Fluor, Chlor, Brom, Hydroxy, Cyano, Perhalogenalkyl mit bis zu 4 Kohlenstoffatomen oder durch geradkettiges oder verzweigtes Acyl, Alkoxy, Vinylalkoxycarbonyl oder Alkoxycarbonyl mit jeweils bis zu 5 Kohlenstoffatomen substituiert ist, das seinerseits durch geradkettiges oder verzweigtes Alkoxy mit bis zu 6 Kohlenstoffatomen substituiert ist, oder
einen Aminosäurerest der Formel

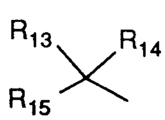

bedeutet,
worin

$R^{13}$ und $R^{14}$ gleich oder verschieden sind und Wasserstoff oder Methyl bedeuten, oder

$R^{13}$ und $R^{14}$ gemeinsam einen Cyclopentyl- oder Cyclohexyl-ring bilden,
oder

$R^{13}$ Wasserstoff oder Methyl bedeutet
und

$R^{14}$ Cyclopropyl, Cyclopentyl, Cyclohexyl, Phenyl oder Wasserstoff bedeutet, oder
geradkettiges oder verzweigtes Alkyl mit bis zu 6 Kohlenstoffatomen bedeutet,
wobei das Alkyl gegebenenfalls durch Methylthio, Hydroxy, Mercapto,

Guanidyl oder durch eine Gruppe der Formel $-NR^{16}R^{17}$ oder $R^{18}$-OC- substituiert ist,
worin

$R^{16}$ und $R^{17}$ unabhängig voneinander Wasserstoff, geradkettiges oder verzweigtes Alkyl mit bis zu 6 Kohlenstoffatomen oder Phenyl bedeuten,
und

$R^{18}$ Hydroxy, Benzyloxy, Alkoxy mit bis zu 6 Kohlenstoffatomen oder die oben aufgeführte Gruppe $-NR^{16}R^{17}$ bedeutet,

oder das Alkyl gegebenenfalls durch Cyclopropyl, Cyclopentyl, Cyclohexyl oder Phenyl substituiert ist, das seinerseits durch Hydroxy, Fluor, Chlor, Brom, Nitro, Alkoxy mit bis zu 8 Kohlenstoffatomen oder durch die Gruppe $-NR^{16}R^{17}$ substituiert ist,
worin

$R^{16}$ und $R^{17}$ die oben angegebene Bedeutung haben,

oder das Alkyl gegebenenfalls durch Imidazolyl oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,

$R^{15}$ geradkettiges oder verzweigtes Alkyl mit bis zu 6 Kohlenstoffatomen bedeutet, das gegebenenfalls durch Hydroxy oder geradkettiges oder verzweigtes Alkoxy mit bis zu 4 Kohlenstoffatomen substituiert ist, oder
Carboxy, Allyloxycarbonyl, geradkettiges oder verzweigtes Alkoxycarbonyl mit bis zu 6 Kohlenstoffatomen oder Benzyloxycarbonyl bedeutet,

$R^7$ einen Rest der Formel

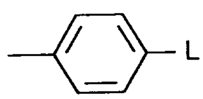

bedeutet,
worin

L Phenyl oder Pyridyl bedeutet

$R^8$ Wasserstoff, geradkettiges oder verzweigtes Alkyl mit bis zu 4 Kohlenstoffatomen, tert.Butoxycarbonyl (Boc) oder Benzyloxycarbonyl (Z) bedeutet,

$R^9$ geradkettiges oder verzweigtes Alkyl mit bis zu 6 Kohlenstoffatomen, welches durch Pyridyl substituiert sein kann, bedeutet, oder Phenoxy, Phenyl, Naphthyl, Indolyl, Chinolyl, Chinoxalinyl, Isochinolyl, Pyridyl, Pyrazinyl, Pyrimidyl, Triazolyl oder Imidazolyl bedeutet, wobei die Cyclen gegebenenfalls bis zu 3-fach gleich oder verschieden durch Nitro, Carboxy, Cyano, Hydroxy, Fluor, Chlor, Brom, Perhalogenalkyl mit bis zu 4 Kohlenstoffatomen oder durch geradkettiges oder verzweigtes Alkyl, Acyl, Alkoxy oder Alkoxycarbonyl mit jeweils bis zu 4 Kohlenstoffatomen substituiert sind, oder
Phenyl gegebenenfalls durch Pyridyl oder Triazolyl substituiert ist, wobei diese wiederum durch Phenyl substituiert sein können,
oder

$R^9$ einen Rest der Formel

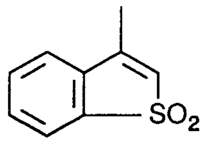 , 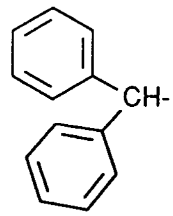 ,

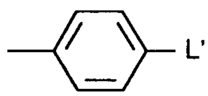 oder 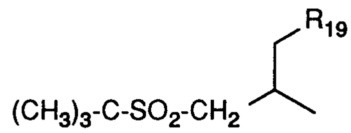

bedeuten,
worin

L' die oben angegebene Bedeutung von L hat und mit dieser gleich oder verschieden ist,

$R^{19}$ Phenyl oder Naphthyl bedeutet,

c eine Zahl 0, 1, 2 oder 3 bedeutet,

d eine Zahl 0, 1 oder 2 bedeutet,

$R^{10}$ die oben angegebene Bedeutung von $R^9$ hat und mit dieser gleich oder verschieden ist,

$R^{11}$ Methyl oder Phenyl bedeutet, das gegebenenfalls bis zu 4-fach gleich oder verschieden durch Methyl oder Methoxy substituiert ist, oder
einen Rest der Formel

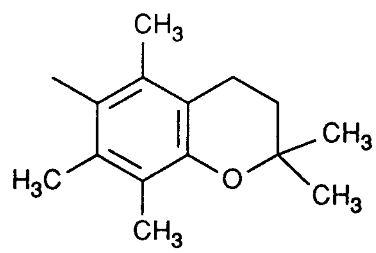

bedeutet,

$R^2$, $R^3$ und $R^5$ gleich oder verschieden sind und Boc, Wasserstoff, Methyl, Ethyl, Benzyloxycarbonyl oder tert.Butyl bedeuten,

$R^4$ für Wasserstoff, Nitro, Benzyloxycarbonyl, tert.Butoxycarbonyl oder für einen Rest der Formel $-SO_2R^{20}$ steht,
worin

$R^{20}$ die oben angegebene Bedeutung von $R^{11}$ hat und mit dieser gleich oder verschieden ist,

A für eine Bindung oder für einen Rest der Formel



steht,
worin

$R^{21}$ und $R^{22}$ gleich oder verschieden sind und die oben angegebene Bedeutung von $R^{13}$ und $R^{14}$ haben und mit dieser gleich oder verschieden sind,

$R^{23}$ die oben angegebene Bedeutung von $R^2$, $R^3$ oder $R^5$ hat und mit dieser gleich oder verschieden ist,

$R^6$ für Formyl oder Carboxy steht oder für geradkettiges oder verzweigtes Alkoxycarbonyl mit bis zu 6 Kohlenstoffatomen steht, oder für einen Rest der Formel $-CH_2-OR^{24}$ oder $-CH(OR^{25})_2$ steht, worin

$R^{24}$ und $R^{25}$ gleich oder verschieden sind und Wasserstoff, geradkettiges oder verzweigtes Alkyl mit bis zu 4 Kohlenstoffatomen, Acetyl oder Benzyl bedeuten,

und deren Salze, mit der Maßgabe, daß A nicht für Valin stehen darf.

**3.** Verbindungen der allgemeinen Formel (I), gemäß Anspruch 1 in welcher

a für eine Zahl 2 oder 3 steht,

b für eine Zahl 0 oder 1 steht,

$R^1$ für Wasserstoff, tert.Butoxycarbonyl (Boc) oder Benzyloxycarbonyl (Z) steht, oder für einen Rest der Formel $R^7-NR^8-CO-$, $R^9-(CH_2)_c-CO-$, $R^{10}-(CH_2)_d-O-CO$ oder für einen Rest der Formel $-SO_2-R^{11}$ stehen, worin

$R^7$ Cyclopentyl oder Cyclohexyl bedeutet oder geradkettiges oder verzweigtes Alkyl mit bis zu 14 Kohlenstoffatomen bedeutet, das gegebenenfalls durch Hydroxy, Methoxy, Fluor, Trifluormethyl, Trifluormethoxy, Cyclohexyl oder Phenyl substituiert ist, das gegebenenfalls durch eine Gruppe der Formel $-CO_2R^{12}$ substituiert ist, worin

$R^{12}$ Wasserstoff oder geradkettiges oder verzweigtes Alkyl oder Alkenyl mit jeweils bis zu 4 Kohlenstoffatomen oder Benzyl bedeutet, oder

$R^7$ Phenyl bedeutet, das gegebenenfalls bis zu 3-fach gleich oder verschieden durch Carboxy, Fluor, Hydroxy, Cyano, Trifluormethyl, Amino oder durch geradkettiges oder verzweigtes Acyl, Alkoxy, Vinylalkoxycarbonyl oder Alkoxycarbonyl mit jeweils bis zu 4 Kohlenstoffatomen substituiert ist, das seinerseits durch geradkettiges oder verzweigtes Alkoxy mit bis zu 4 Kohlenstoffatomen substituiert ist, oder einen Aminosäurerest der Formel

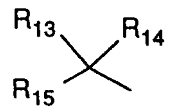

bedeutet, worin

$R^{13}$ und $R^{14}$ gleich oder verschieden sind und Wasserstoff oder Methyl bedeuten, oder

$R^{13}$ und $R^{14}$ gemeinsam einen Cyclopentyl- oder Cyclohexylring bilden, oder

$R^{13}$ Wasserstoff oder Methyl bedeutet und

$R^{14}$ Cyclopropyl, Cyclopentyl, Cyclohexyl, Phenyl oder Wasserstoff bedeutet, oder geradkettiges oder verzweigtes Alkyl mit bis zu 6 Kohlenstoffatomen bedeutet, wobei das Alkyl gegebenenfalls durch Methylthio, Hydroxy, Mercapto, Guanidyl oder durch eine Gruppe der Formel $-NR^{16}R^{17}$ oder $R^{18}-OC-$ substituiert ist, worin

$R^{16}$ und $R^{17}$ unabhängig voneinander Wasserstoff, geradkettiges oder verzweigtes Alkyl mit bis zu 6 Kohlenstoffatomen oder Phenyl bedeuten, und

$R^{18}$ Hydroxy, Benzyloxy, Alkoxy mit bis zu 4 Kohlenstoffatomen oder die oben

aufgeführte Gruppe $-NR^{16}R^{17}$ bedeutet,

oder das Alkyl gegebenenfalls durch Cyclopropyl, Cyclopentyl, Cyclohexyl oder Phenyl substituiert ist, das seinerseits durch Hydroxy, Fluor, Chlor, Brom, Nitro, Alkoxy mit bis zu 6 Kohlenstoffatomen oder durch die Gruppe $-NR^{16}R^{17}$ substituiert ist,

worin

$R^{16}$ und $R^{17}$ die oben angegebene Bedeutung haben,

oder das Alkyl gegebenenfalls durch Imidazolyl oder Indolyl substituiert ist, worin die entsprechenden -NH-Funktionen gegebenenfalls durch Alkyl mit bis zu 4 Kohlenstoffatomen, tert. Butoxycarbonyl oder Benzyloxycarbonyl geschützt sind,

$R^{15}$ geradkettiges oder verzweigtes Alkyl mit bis zu 4 Kohlenstoffatomen bedeutet, das gegebenenfalsl durch Hydroxy oder geradkettiges oder verzweigtes Alkoxy mit bis zu 3 Kohlenstoffatomen substituiert ist, oder

Carboxy, Allyloxycarbonyl, geradkettiges oder verzweigtes Alkoxycarbonyl mit bis zu 4 Kohlenstoffatomen oder Benzyloxycarbonyl bedeutet,

$R^7$ einen Rest der Formel

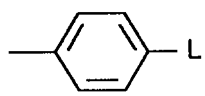

bedeutet,

worin

L Phenyl oder Pyridyl bedeutet

$R^8$ Wasserstoff, Methyl, Ethyl oder tert.Butyl, bedeutet,

$R^9$ geradkettiges oder verzweigtes Alkyl mit bis zu 6 Kohlenstoffatomen, welches durch Pyridyl substituiert sein kann, Phenoxy, Phenyl, Naphthyl, Indolyl, Chinolyl, Chinoxalinyl, Isochinolyl, Pyridyl, Pyrazinyl, Pyrimidyl, Triazolyl oder Imidazolyl bedeutet, wobei die Cyclen gegebenenfalls bis zu 3-fach gleich oder verschieden durch Nitro, Carboxy, Cyano, Hydroxy, Fluor, Chlor, Brom, Perhalogenalkyl mit bis zu 4 Kohlenstoffatomen oder durch geradkettiges oder verzweigtes Alkyl, Acyl, Alkoxy oder Alkoxycarbonyl mit jeweils bis zu 4 Kohlenstoffatomen substituiert sind, oder

Phenyl gegebenenfalls durch Pyridyl oder riazolyl substituiert ist, wobei diese wiederum durch Phenyl substituiert sein können,

oder

$R^9$ einen Rest der Formel

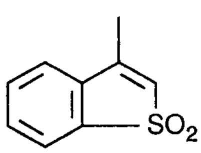 , 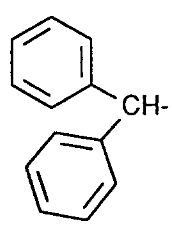 ,

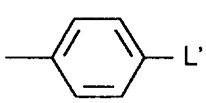 oder 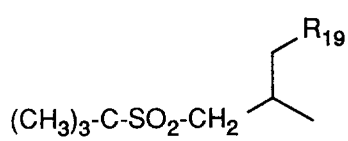

bedeutet,

worin

L' die oben angegebene Bedeutung von L hat und mit dieser gleich oder verschieden ist,

| | |
|---|---|
| $R^{19}$ | Phenyl oder Naphthyl bedeutet, |
| c | eine Zahl 0, 1, 2 oder 3 bedeutet, |
| d | eine Zahl 0, 1 oder 2 bedeutet, |
| $R^{10}$ | die oben angegebene Bedeutung von $R^9$ hat und mit dieser gleich oder verschieden ist, |
| $R^{11}$ | Methyl oder Phenyl bedeutet, das gegebenenfalls bis zu 4-fach gleich oder verschieden durch Methyl oder Methoxy substitiert ist, oder einen Rest der Formel |

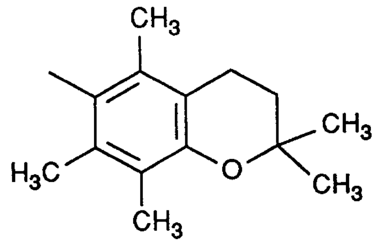

| | |
|---|---|
| | bedeutet, |
| $R^2$, $R^3$ und $R^5$ | gleich oder verschieden sind und Boc, Wasserstoff, Methyl, Ethyl, Benzyloxycarbonyl oder tert.Butyl bedeuten, |
| $R^4$ | für Wasserstoff, Nitro, Benzyloxycarbonyl, tert.Butoxycarbonyl oder für einen Rest der Formel $-SO_2R^{20}$ steht, |
| | worin |
| $R^{20}$ | die oben angegebene Bedeutung von $R^{11}$ hat und mit dieser gleich oder verschieden ist, |
| A | für eine Bindung oder für einen Rest der Formel |

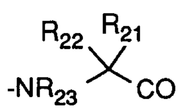

| | |
|---|---|
| | steht, |
| | worin |
| $R^{21}$ und $R^{22}$ | gleich oder verschieden sind und die oben angegebene Bedeutung von $R^{13}$ und $R^{14}$ haben und mit dieser gleich oder verschieden sind, |
| $R^{23}$ | die oben angegebene Bedeutung von $R^2$, $R^3$ oder $R^5$ hat und mit dieser gleich oder verschieden ist, |
| $R^6$ | für Formyl oder Carboxy steht oder<br>für geradkettiges oder verzweigtes Alkoxycarbonyl mit bis zu 4 Kohlenstoffatomen steht, oder<br>für einen Rest der Formel $-CH_2-OR^{24}$ oder $-CH(OR^{25})_2$ steht,<br>worin |
| $R^{24}$ und $R^{25}$ | gleich oder verschieden sind und Wasserstoff, geradkettiges oder verzweigtes Alkyl mit bis zu 3 Kohlenstoffatomen oder Benzyl bedeuten, |

und deren Salze,
mit der Maßgabe, daß A nicht für Valin stehen darf.

**4.** Verfahren zur Herstellung der Verbindungen der allgemeinen Formel (I) gemäß Anspruch 1, dadurch gekennzeichnet, daß man Verbindungen der allgemeinen Formel (III)

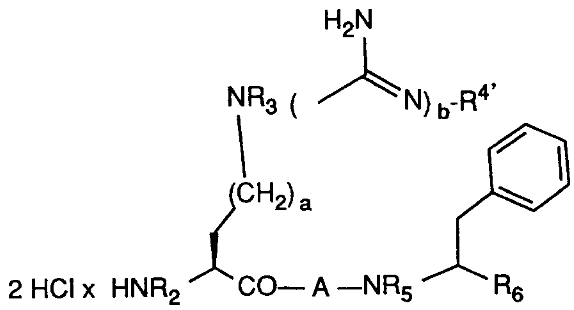

(III)

in welcher

A, a, b, $R^2$, $R^3$, $R^5$ und $R^6$ die in Anspruch 1 bis 3 angegebene Bedeutung haben,

und

$R^{4'}$ die in den Ansprüchen 1 bis 3 angegebene Bedeutung von $R^4$ hat, aber nicht für Wasserstoff steht,

[A] im Fall, daß $R^1$ für den Rest der Formel $R^7$-$NR^8$-CO- steht, zunächst durch Umsetzung mit Verbindungen der allgemeinen Formel (IV)

$R^7$-N=C=O (IV)

in welcher

$R^7$ die in den Ansprüchen 1 bis 3 angegebene Bedeutung hat,

in inerten Lösemitteln, in Anwesenheit einer Base, in die Verbindungen der allgemeinen Formel (V)

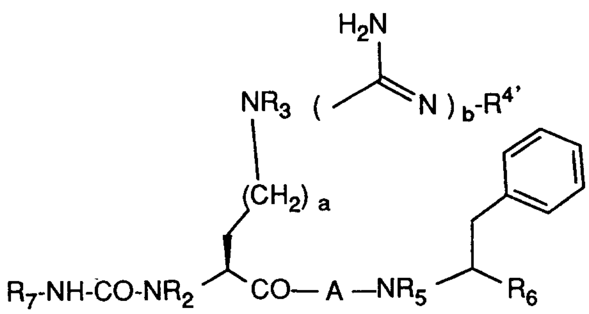

(V)

in welcher

A, a, b, $R^1$, $R^2$, $R^3$, $R^5$, $R^6$ und $R^7$ die in den Ansprüchen 1 bis 3 angegebene Bedeutung haben,

überführt,

oder

[B] im Fall, daß $R^1 \neq R^7$-NH-CO- Verbindungen der allgemeinen Formel (III) mit Verbindungen der allgemeinen Formel (VI) oder (VII)

V-CO-W (VI) oder X-$SO_2$-$R^{11}$ (VII)

in welcher

$R^{11}$ die in den Ansprüchen 1 bis 3 angegebene Bedeutung hat,

V den in den Ansprüchen 1 bis 3 aufgeführten Bedeutungsumfang der Reste $R^9$-$(CH_2)_c$ oder $R^{10}$-$(CH_2)_d$-O- umfaßt

und W und X gleich oder verschieden sind und Hydroxy oder einen typischen carbonsäureaktivierenden Rest, wie beispielsweise Chlor, bedeuten,

nach denen, in der Peptidchemie üblichen Methoden, in inerten organischen Lösemitteln und in Anwesenheit einer Base und eines Hilfsstoffes umsetzt,

und im Fall, daß $R^2$, $R^3$, $R^5$ und $R^8 \neq$ H gegebenenfalls eine Alkylierung nach üblichen Methoden anschließt,

und im Fall, $R^6$ = $CH_2$-OH, die Verbidugen der allgemeinen Formel (V) ($R^6$ = $COOCH_3$) nach üblichen Methoden, vorzugsweise aber mit Natriumborhydrid umsetzt,
und im Fall, $R^6$ = CHO, die Verbindungen der allgemeinen Formel (V), ausgehend von der Hydroxymethylverbindung ($R^6$ = $CH_2$-OH) einer Oxidation unterzieht,
in Abhängigkeit des Restes $R^{4'}$, beispielsweise mit Fluorwasserstoffsäure oder Trifluoressigsäure zu $R^4$ = H umsetzt,
und im Fall einer Aminoschutzgruppe ($R^1$, $R^2$, $R^3$, $R^{4'}$ und $R^5$) diese nach denen in der Peptidchemie üblichen Methoden abspaltet,
und im Fall der Säuren die Ester verseift.

**5.** Arzneimittel enthaltend eine oder mehrere Verbindungen der Ansprüche 1 bis 3.

**6.** Verwendung der Verbndungen gemäß den Ansprüchen 1 bis 3 zur Herstellung von Arzneimitteln.

Europäisches Patentamt

# EUROPÄISCHER RECHERCHENBERICHT

Nummer der Anmeldung

EP 94113560.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.6) |
|---|---|---|---|
| P,A | EP - A - 0 611 776 (BAYER AG) * Ansprüche 1,4-7 * | 1-6 | C 07 K 5/068<br>A 61 K 38/02 |
| A | US - A - 5 086 069 (KLEIN et al.) * Ansprüche 1,2 * | 1-6 | |
| A | CHEMICAL ABSTRACTS, Band 115, Nr. 9, 2. September 1991, Columbus, Ohio, USA M. RAJU et al. "Synthesis and biological activity of angiotensin II analog containing a Val-His replacement, Val-(CH(CONH2) HN)His" Seite 831, Nr. 92 899n; & J.hed. Chem. 1991 34(8), 2402-10 | 1-6 | |
| A | CHEMICAL ABSTRACTS, Band 114, Nr. 17, 29. April 1991, Columbus, Ohio, USA P.E. REED et al. "Synthesis of proline-valine psendodipeptide enol lactones serine protease inhibitors" Seite 834, Nr. 164 760w; & J. Org. Chem. 1991, 56(8), 2624-34 | 1-6 | RECHERCHIERTE SACHGEBIETE (Int. Cl6)<br>C 07 K 5/00<br>A 61 K 38/00 |

Der vorliegende Recherchenbericht wurde für alle Patentansprüche erstellt.

| Recherchenort | Abschlußdatum der Recherche | Prüfer |
|---|---|---|
| WIEN | 12-11-1994 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument